# EUROPÄISCHE PATENTSCHRIFT

(11) **EP 0 674 880 B1**
(45) Veröffentlichungstag und Bekanntmachung des Hinweises auf die Patenterteilung: **11.12.1996**
(21) Anmeldenummer: 95200700.3
(22) Anmeldetag: 22.03.1995
(51) Int. Cl.: A61B 17/58, F16B 23/00, A61B 17/86

(54) **Schraube aus bioabbaubarem Material für osteochirurgische Zwecke, sowie dazu passender Schraubendreher**
Biodegradable screen for bone surgery, and corresponding screwdriver
Vis biodégradable pour la chirurgie osseuse, et tournevis correspondant

(30) Priorität: 29.03.1994 CH 938/94; 12.12.1994 CH 3757/94
(43) Veröffentlichungstag der Anmeldung: 04.10.1995
(73) Patentinhaber: Stähelin, Andreas C., Dr. med., CH-4052 Basel (CH)
(72) Erfinder: Stähelin, Andreas C., Dr. med., CH-4052 Basel (CH)
(74) Vertreter: Köver, François

(56) Entgegenhaltungen:
- EP-A- 0 502 698
- DE-U- 8 804 456
- GB-A- 2 022 482
- US-A- 4 146 073
- US-A- 5 269 209
- Handbuch der Mathematik; Buch und Zeit Verlagsges.m.b.H. Köln; Seite 178
- Schülerduden "Die Mathematik"; Bibliographisches Institut AG, Mannheim 1982; Seiten 433, 518-521

## Beschreibung

Die Erfindung betrifft eine Schraube aus bioabbaubarem Material für osteochirurgische Zwecke mit einem länglichen Schraubenkörper, der sich in Richtung einer Längsachse davon zwischen einem proximalen und einem distalen Ende davon erstreckt, wobei
eine Aussenfläche des Schraubenkörpers mit einem zur Längsachse koaxialen Aussengewinde versehen ist, das zum Führen und Halten der Schraube an Knochenmaterial eines Patienten bestimmt ist und eine Drehrichtung um die Längsachse für das Einschrauben des Schraubenkörpers und Vorrücken des distalen Endes in das Knochenmaterial definiert,
im Schraubenkörper ein länglicher, zur Längsachse koaxialer Kanal vorgesehen ist, der am proximalen Ende zur Aufnahme eines Schafts eines zum Drehen der Schraube bestimmten Schraubendrehers offen ist,
der Kanal eine Kanalwand aufweist, deren Form um die Längsachse in diskreten regelmässigen Drehschritten radialsymmetrisch ist und in einem Querschnitt des Schraubenkörpers rechtwinklig zur Längsachse eine Spur ergibt, welche eine um eine Spur der Längsachse in diskreten regelmässigen Drehschritten radialsymmetrische geschlossene Umrisslinie ist,
die Kanalwand einen axialen Kanalbereich und eine Mehrzahl von um den Kanalbereich regelmässig angeordneten Lappenbereichen definiert, so dass der Kanal im wesentlichen aus der Vereinigung des axialen Kanalbereiches und der Lappenbereiche besteht, und
in jedem Lappenbereich die Kanalwand ein Paar von Flanken aufweist, die sich im wesentlichen vom Kanalbereich bis in Nähe eines dem Kanalbereich abgewandten Endes des Lappenbereiches erstrecken und unter denen mit Bezug auf die genannte Drehrichtung jeweils eine vorlaufende Flanke und eine nachlaufende Flanke definiert ist.

Ebenfalls betrifft die Erfindung einen Schraubendreher zum Eindrehen dieser Schraube in Knochenmaterial eines Patienten während eines osteochirurgischen Eingriffs, wobei der Schraubendreher mit einem Griff und einem daran befestigten länglichen Schaft versehen und der Schaft in den Kanal der Schraube einführbar und daraus entfernbar ist.

Eine Schraube der eingangs genannten Art ist beispielsweise aus EP-A-0502698 bekannt.

Das eingangs genannte bioabbaubare, d.h. im Körper eines Patienten resorbierbare Material der Schraube ist aus EP-A-0502698 wie auch aus DE-A-2742128 und EP-A-0011528 bekannt. Im wesentlichen handelt es sich bei diesem Schraubenmaterial um ein Polylactid, ein Polyglycolid oder ein Copolymer davon, das ausgezeichnet formbar, sterilisierbar und resorbierbar ist.

Dieses Schraubenmaterial ist jedoch auch spröde, was bei der Anwendung der Schraube zu Problemen führt. Beim Eindrehen der Schraube in das Knochenmaterial eines Patienten während eines osteochirurgischen Eingriffs überträgt der Schraubendreher auf die Schraube Kräfte, die das spröde Schraubenmaterial übermässig beanspruchen können, mit der Folge, dass die Schraube beschädigt wird. Beispielsweise besteht bei hartem Knochen die Gefahr, dass der Schraubendreher in der Schraube durchdreht.

Zur Lösung der angesprochenen Probleme ist bereits eine Vielzahl von sternförmigen Ausbildungen des Kanals bzw. dessen Spur im Querschnitt des Schraubenkörpers vorgeschlagen worden. Eben diese Vielzahl von Formen zeigt aber auf, dass sich noch keine bisherige Schraube als zufriedenstellend erwies. Bei den bekannten Formen des Kanals sind die aneinander anliegenden, Kraft übertragenden Flanken der Lappenbereiche der Schraube und des Schraubendrehers so orientiert, dass an diesen Flanken beim Eindrehen der Schraube in Knochenmaterial radial nach aussen gerichtete Komponenten der Kraft entstehen. Auf den Körper der Schraube wirken deshalb Expansionskräfte, welche die Schraube aufzusprengen tendieren.

Aufgabe der Erfindung ist es demnach, eine Schraube der eingangs genannten Art und einen dazu passenden Schraubendreher vorzuschlagen, mit denen die erwähnten Nachteile der bekannten Schrauben und der dazu passenden Schraubendreher überwunden werden.

Zur Lösung dieser Aufgabe geht die Erfindung von der Erkenntnis aus, dass Kräfte auf die Schraube nur in Richtung des Eindrehens auszuüben sind. Bei eine osteochirurgischen Eingriff braucht eine gut sitzende Schraube der eingangs genannten Art kaum jemals entfernt zu werden. Ist eine solche Schraube zu entfernen, so ist es im allgemeinen, weil sie nicht gut sitzt, wobei sie dann eben locker sitzt und folglich keine nennenwerten Kräfte in Gegenrichtung zum Eindrehen darauf auszuüben sind, um sie wegzuschrauben. Daher braucht die Schraube gar nicht, wie bisher beim Stand der Technik, in bezug auf eine durch ihre Längsachse gehende diametrale Ebene symmetrisch zu sein.

Aufgrund dieser Erkenntnis wird die genannte Aufgabe bei einer Schraube der eingangs genannten Art erfindungsgemäss dadurch gelöst, dass der Kanal sich bis in den Bereich des distalen Endes erstreckt und, in einem Querschnitt des Schraubenkörpers rechtwinklig zur Längsachse betrachtet, jeder Lappenbereich in bezug auf eine durch die Längsachse gehende diametrale Ebene asymmetrisch ausgebildet ist, wobei ein der vorlaufenden Flanke entsprechender Abschnitt der Umrisslinie kürzer ist als ein der nachlaufenden Flanke entsprechender Abschnitt der Umrisslinie, so dass der Lappenbereich beim Verlauf des radialen Abstands von der Längsachse entlang der Umrisslinie ein sägezahnähnliches Profil mit durchschnittlich steilerem Anstieg der vorlaufenden Flanke und flacherem Abfall der nachlaufenden Flanke aufweist.

Ein erfindungsgemässer Schraubendreher weist die Merkmale des Anspruchs 18 auf, wobei sein Schaft eine zum Kanal der Schraube komplementär passende Form aufweist.

Bei der erfindungsgemässen Schraube ist die Übertragung der Kraft vom erfindungsgemässen Schraubendreher auf die Schraube insofern optimal, als der Schraubendreher in der Schraube auch bei grossen Drehmomenten nicht durchdreht. Auf den Körper der Schraube aus sprödem Material wirken Expansionskräfte, die geringer sind als bisher beim Stand der Technik mit radialsymmetrischen Schrauben, was dem Material der Schraube optimal angepasst ist. Dadurch verträgt eine erfindungsgemässe Schraube bei ihrem Einschrauben in Knochenmaterial grössere Drehmomente, als es mit den bisherigen Schrauben zulässig war. Der Körper der Schraube braucht nicht massiv zu sein, um die Drehmomente zu vertragen, deshalb kann sich der Kanal ganz in die Nähe vom und gegebenenfalls ganz bis zum distalen Ende erstrecken, was wiederum erlaubt, die Drehmomente auf die Schraube gleichmässig, im wesentlichen über deren ganze Länge zu übertragen und zu verteilen.

In vielen Fällen wird somit auch bei hartem Knochen möglich, auf das Vorschneiden eines Gewindes im Knochen zu verzichten, was den Arbeitsablauf eines osteochirurgischen Eingriffs wesentlich vereinfacht.

Die nachstehend angegebenen bevorzugten Ausbildungen gewährleisten in verschiedenen Varianten eine optimale Orientierung bzw. Umlenkung der vom Schraubendreher auf die Schraube ausgeübten Kräfte, so dass auf den Schraubenkörper keine Expansionskräfte, sondern nur Zug und Schub wirken.

In einer bevorzugten Ausbildung einer erfindungsgemässen Schraube erstreckt sich der Kanal bis in den Bereich des distalen Endes, und in jedem Lappenbereich weist mindestens eine der genannten Flanken zumindest in Nähe des genannten Endes des Lappenbereiches eine mit wachsendem radialem Abstand von der Längsachse in der genannten Drehrichtung zunehmend vorlaufende Form auf.

Bei dieser Ausbildung der erfindungsgemässen Schraube wirken auf den Körper der Schraube aus sprödem Material keine Expansionskräfte, sondern nur Zug und Schub.

In einer bevorzugten Ausbildung sind, in jedem Lappenbereich, die beiden Flanken in einem Abstand voneinander, der sich mit wachsendem radialem Abstand von der Längsachse zum Ende des Lappenbereiches hin verjüngt. Vorzugsweise können dabei die beiden Flanken am Ende des Lappenbereiches zu einer Kante zusammenlaufen.

In einer weiteren bevorzugten Ausbildung ist in jedem Lappenbereich die vorlaufende Flanke zumindest in Nähe des Endes des Lappenbereiches zur genannten Drehrichtung hin konvex gekrümmt, oder sie liegt im wesentlichen in einer durch die Längsachse gehende diametrale Ebene.

In einer noch weiteren bevorzugten Ausbildung ist in jedem Lappenbereich die vorlaufende Flanke zumindest in Nähe des Endes des Lappenbereiches zur genannten Drehrichtung hin konkav gekrümmt.

In einer noch weiteren bevorzugten Ausbildung ist die nachlaufende Flanke zumindest in Nähe des Endes des Lappenbereiches entgegengesetzt zur genannten Drehrichtung konvex gekrümmt.

In einer bevorzugten Kombination der vorangehend genannten Ausbildungen kann, in einem Querschnitt des Schraubenkörpers rechtwinklig zur Längsachse, die Umrisslinie im wesentlichen einem um die Spur der Längsachse radialsymmetrischen sternförmigen Linienzug mit 3 bis 8 und vorzugsweise 6 zur genannten Drehrichtung hin vorgezogenen, gegebenenfalls sichelförmigen Zacken entsprechen.

In einer anderen, auch bevorzugten Kombination der vorangehend genannten Ausbildungen kann, in einem Querschnitt des Schraubenkörpers rechtwinklig zur Längsachse, die Umrisslinie im wesentlichen einem um die Spur der Längsachse radialsymmetrischen schaufelradförmigen Linienzug mit 3 bis 8 und vorzugsweise 6 zur genannten Drehrichtung hin vorgezogenen, gegebenenfalls etwa sichelförmigen Lappen entsprechen.

In einer weiteren bevorzugten Ausbildung sind in jedem Lappenbereich die beiden Flanken in etwa gleichbleibendem Abstand voneinander. Vorzugsweise kann dabei, in einem Querschnitt des Schraubenkörpers rechtwinklig zur Längsachse, die Umrisslinie im wesentlichen einem um die Spur der Längsachse radialsymmetrischen schaufelradförmigen Linienzug mit 3 bis 8 und vorzugsweise 6 zur genannten Drehrichtung hin vorgezogenen, etwa ringsegmentförmigen Lappen von etwa gleichbleibender Breite entsprechen.

In einer noch weiteren bevorzugten Ausbildung ist in jedem Lappenbereich in einem Querschnitt des Schraubenkörpers rechtwinklig zur Längsachse eine Spur der vorlaufenden Flanke im wesentlichen geradlinig und zur genannten Drehrichtung hin geneigt. Vorzugsweise kann dabei die Neigung der vorlaufenden Flanke bis zu 10° und vorzugsweise etwa 5° betragen.

In einer ersten bevorzugten Weiterbildung einer der vorangehend genannten Ausbildungen sind die Flanken schraubenförmig um die Längsachse zur genannten Drehrichtung hin verdrillt, derart, dass in zwei voneinander verschiedenen Querschnitten des Schraubenkörpers rechtwinklig zur Längsachse die Umrisslinien Kongruenzabbildungen voneinander darstellen, die durch reine Drehung um die Spur der Längsachse zur Kongruenz bringbar sind. Auf diese Weise wird zwischen dem Schraubenkörper und dem Schaft des Schraubendrehers eine verdrillte Kontaktfläche gebildet, welche die vom Schraubendreher auf die Schraube ausgeübten Drehkräfte so umlenkt, dass die erfindungsgemässe Schraube beim Einschrauben noch grössere Drehmomente verträgt als bei einer geraden axialen Ausführung der Kontaktfläche.

In einer zweiten bevorzugten Weiterbildung einer der vorangehend genannten Ausbildungen sind die Flanken in Richtung vom proximalen zum distalen Ende hin verjüngt, derart, dass in zwei voneinander verschiedenen Querschnitten des Schraubenkörpers rechtwinklig zur Längsachse die Umrisslinien Ähnlichkeitsabbildungen voneinander darstellen, die durch zentrische Streckung um die Spur der Längsachse zur Kongruenz bringbar sind. Dadurch kann der Schaft des Schraubendrehers so bemessen werden, dass er ohne Spiel in den Kanal der Schraube passt und dennoch leicht aus dem Schraubenkörper herausgezogen werden kann, was insbesondere bei einer Ausbildung mit der im vorangehenden erwähnten verdrillten Kontaktfläche von grossem Nutzen ist. Wenn dann auch noch vorgesehen wird, dass sich der Kanal nicht ganz bis zum distalen Ende des Schraubenkörpers erstreckt, so füllt der Schaft des in die Schraube eingesetzten Schraubendrehers den Kanal des Schraubenkörpers nicht nur im Querschnitt rechtwinklig zur Längsachse genau, sondern auch in Richtung der Längsachse, bis das freie Ende des Schraubendrehers am distalen Abschluss des Kanals anliegt, wodurch mit Sicherheit vermieden wird, dass der Schaft des Schraubendrehers den Schraubenkörper wie einen Dübel aufspreizt und dabei das spröde Schraubenmaterial sprengt.

In einer bevorzugten Kombination der genannten ersten und zweiten Weiterbildungen sind die Flanken sowohl verdrillt als auch verjüngt, derart, dass in zwei voneinander verschiedenen Querschnitten des Schraubenkörpers rechtwinklig zur Längsachse die Umrisslinien Ähnlichkeitsabbildungen voneinander darstellen, die durch eine Kombination von reiner Drehung und zentrischer Streckung um die Spur der Längsachse zur Kongruenz bringbar sind.

Vorzugsweise entspricht die Verjüngung in Richtung vom proximalen zum distalen Ende hin einem halben Öffnungswinkel von bis zu 10° und vorzugsweise von etwa 2°. Bei einer solchen Bemessung der Verjüngung wird eine Sprengung des Schraubenkörpers durch den Schaft des Schraubendrehers auch dann vermieden, wenn der Kanal sich durchgehend bis zum distalen Ende des Schraubenkörpers erstreckt und dort offen ist, oder auch wenn der distale Abschluss des Kanals unter dem Druck des Schafts des Schraubendrehers nachgibt.

Nachstehend werden Ausbildungsbeispiele der Erfindung anhand der Zeichnung näher erläutert. Es zeigen:
- Fig. 1A bis 1H: eine erste bis achte Ausbildung einer erfindungsgemässen Schraube, in Seitenansicht;
- Fig. 2A bis 2H: die Ausbildungen der erfindungsgemässen Schraube gemäss den entsprechenden Fig. 1A bis 1H, im Längsschnitt und mit einem darin eingesetzten, gebrochen dargestellten erfindungsgemässen Schraubendreher;
- Fig. 3 bis 10: je einen Querschnitt der erfindungsgemässen Schraube entsprechend der Linie A-A der Fig. 1A bis 1H zur Veranschaulichung von jeweiligen Ausbildungen einer Form einer Kanalwand der erfindungsgemässen Schraube;
- Fig. 11 und 12: die jeweils gleiche Darstellung wie in Fig. 9 und 10, mit Einfügung nur einiger Bezugszeichen und hinzugefügter Bemessung eines Winkels; und
- Fig. 13 bis 16: je einen Querschnitt der erfindungsgemässen Schraube entsprechend der Linie A-A der Fig. 1A bis 1H zur Veranschaulichung von jeweiligen Ausbildungen einer Form einer Kanalwand der erfindungsgemässen Schraube.

Die in den Figuren dargestellten erfindungsgemässen Schrauben sind aus einem bioabbaubaren Material für osteochirurgische Zwecke beispielsweise hergestellt. Als Beispiel eines solchen Materials wird hier das im Handel erhältliche und von der Firma ArgoMedical AG, CH-6330 Cham, unter dem Warenzeichen PHUSILINE vertriebene Material angegeben, was jedoch andere bioabbaubaren Materialien nicht ausschliesst. Die Verarbeitung dieses bioabbaubaren Material zur gewünschten Form erfolgt auf bekannte Weise beispielsweise durch Spritzguss, Heissverformung, Heisspressen und ähnlichen Verfahren bei einer dazu geeigneten Temperatur, die meist im Bereich zwischen Raumtemperatur und 100°C auszuwählen ist.

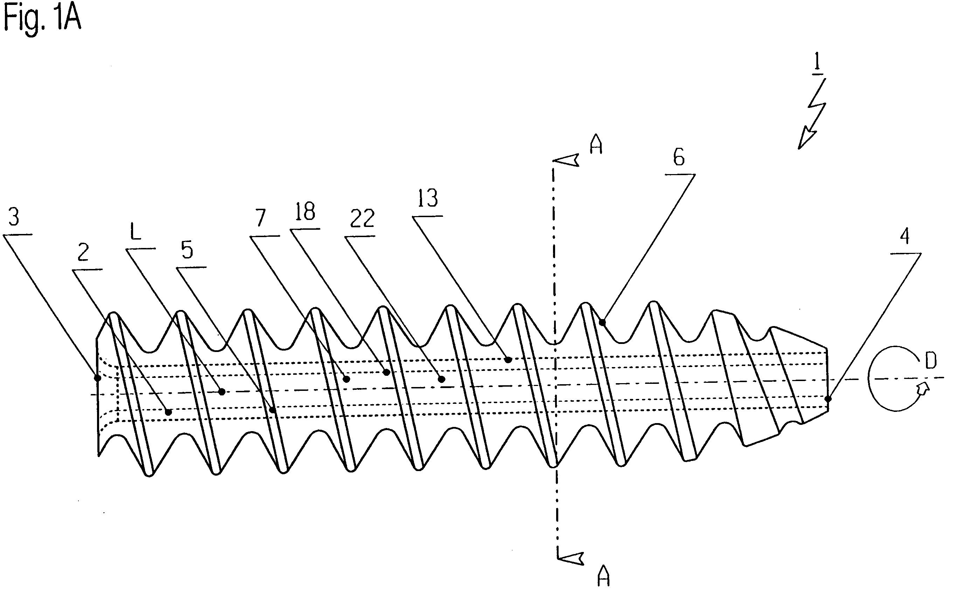

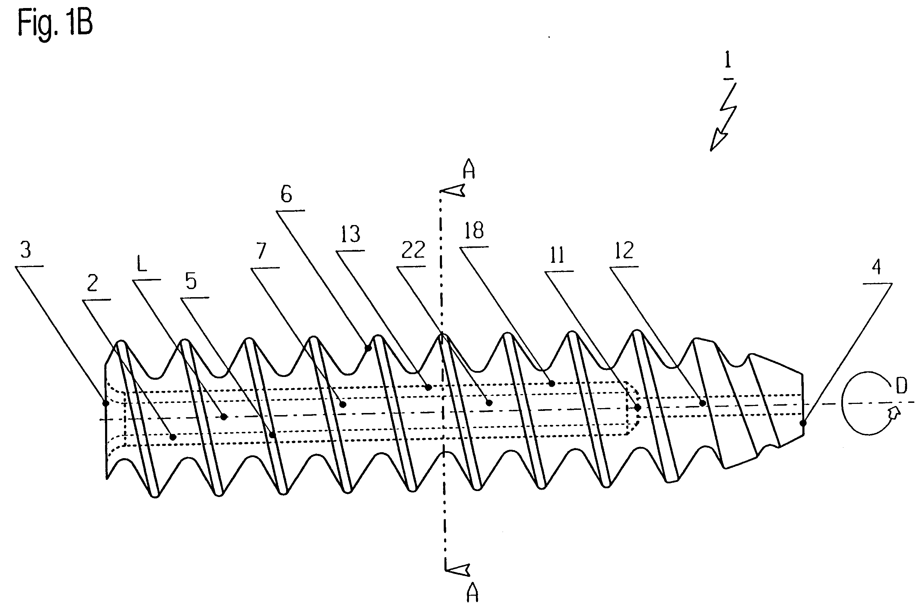

In Fig. 1A bis 1H ist eine Schraube 1 in Seitenansicht dargestellt. Ein Schraubenkörper 2 der Schraube 1 erstreckt sich in Richtung einer Längsachse L der Schraube 1 zwischen einem proximalen Ende 3 und einem distalen Ende 4 der Schraube 1 bzw. des Schraubenkörpers 2. Eine Aussenfläche 5 des Schraubenkörpers 2 ist mit einem zur Längsachse koaxialen Aussengewinde 6 versehen, das zum Führen und Halten der Schraube 1 an Knochenmaterial eines Patienten bestimmt ist. Die Steigung des Aussengewindes 6 definiert eine Drehrichtung D um die Längsachse L, bei welcher die Schraube bzw. der Schraubenkörper 2 eingeschraubt wird, wobei das distale Ende 4 des Schraubenkörpers 2 in das Knochenmaterial vorrückt.

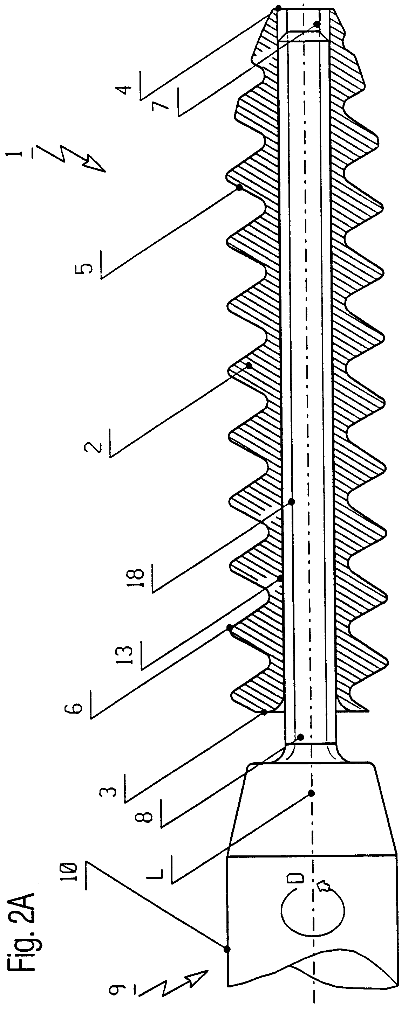

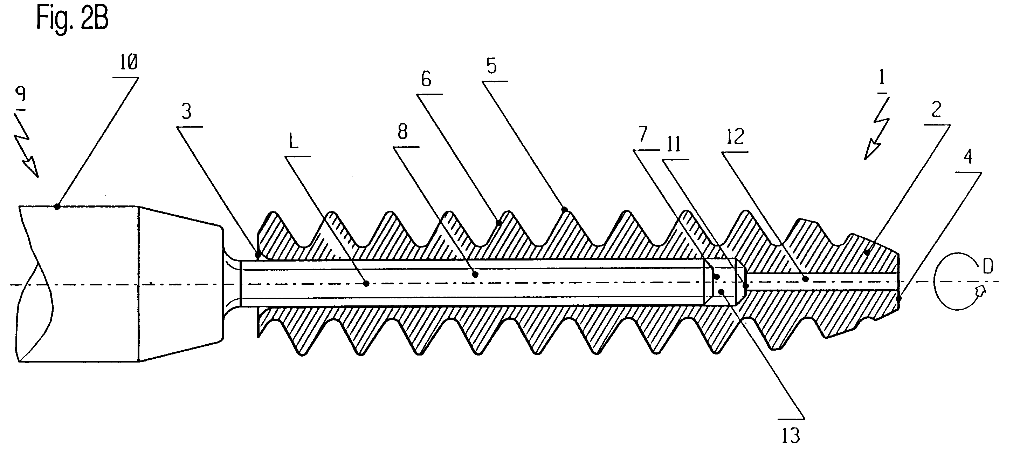

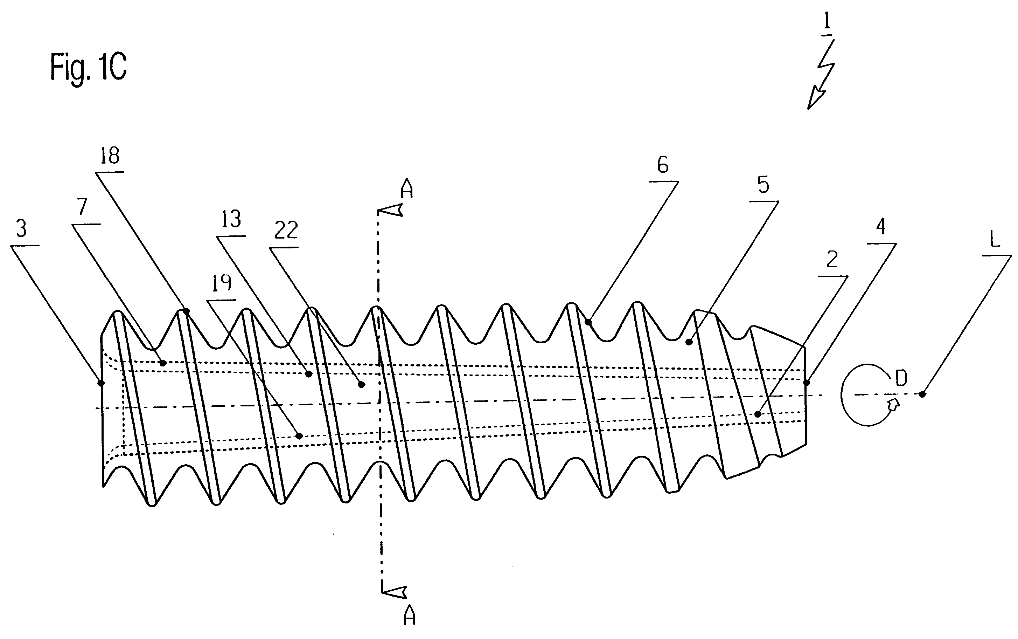

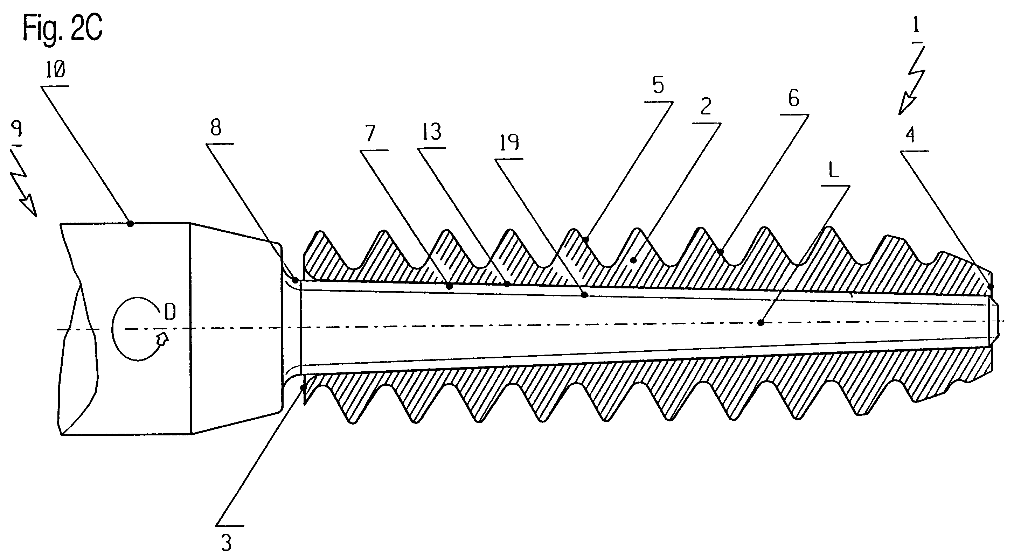

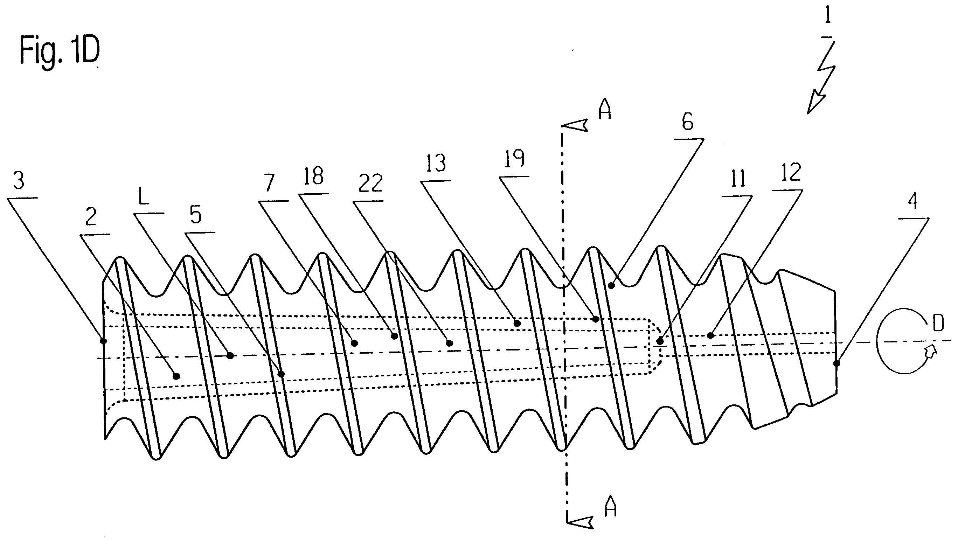

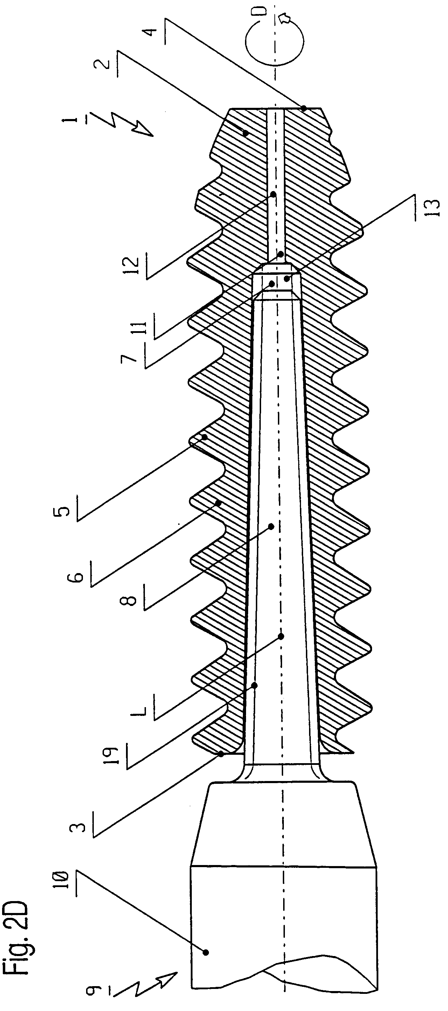

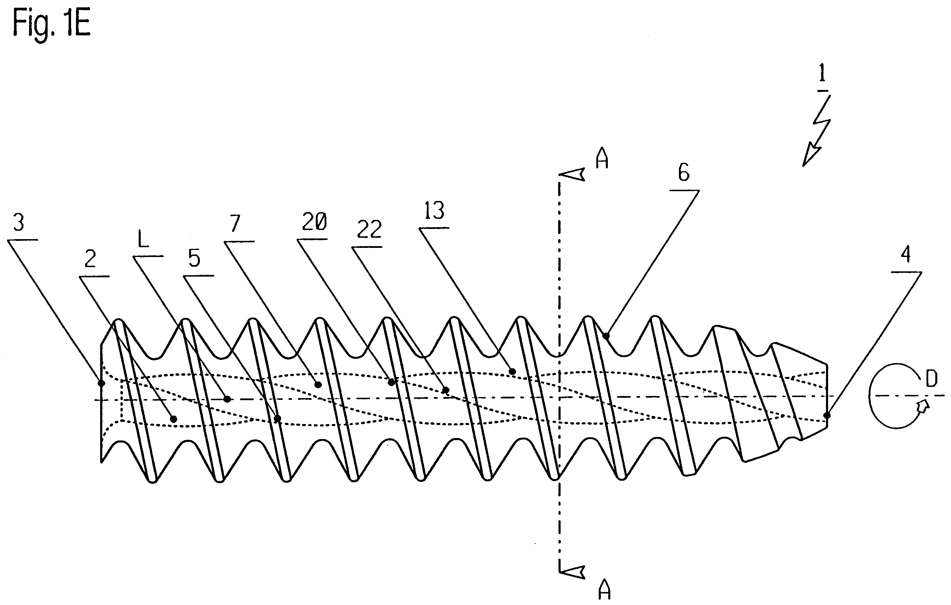

Je eine Schraube 1 der vorangehend beschriebenen Art ist in Fig. 2A bis 2H im Längsschnitt dargestellt.

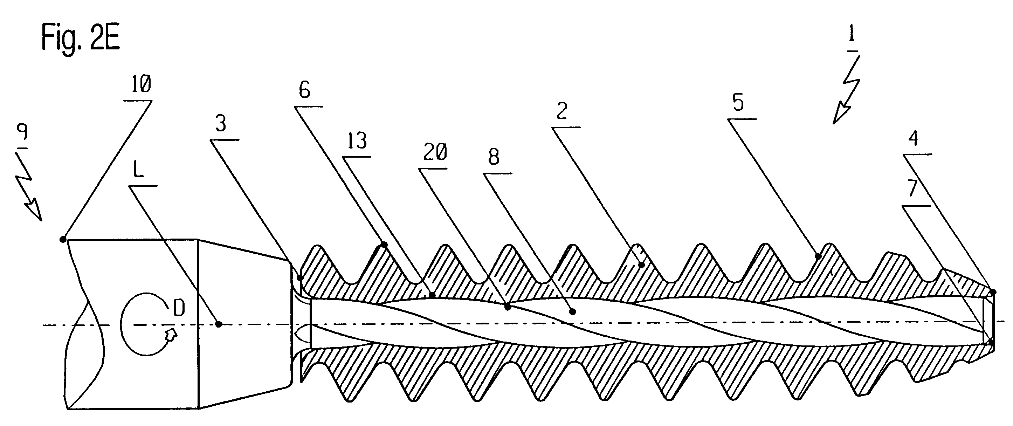

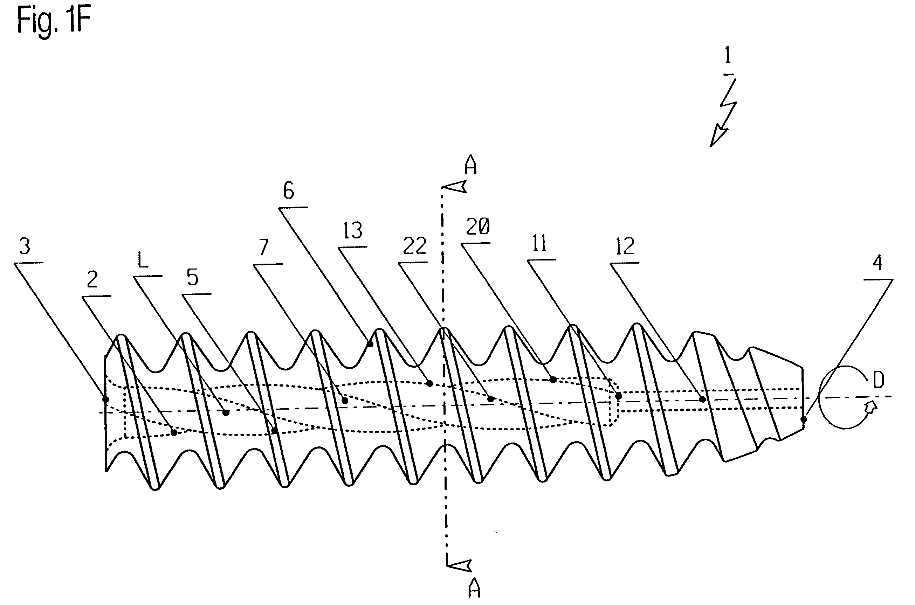

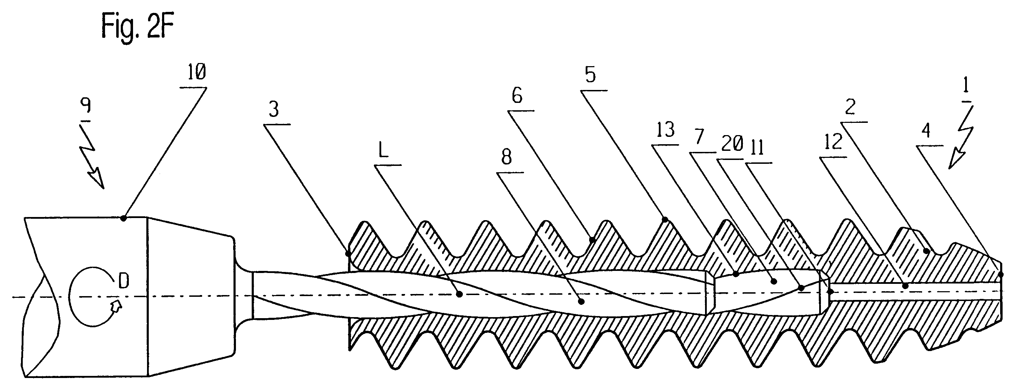

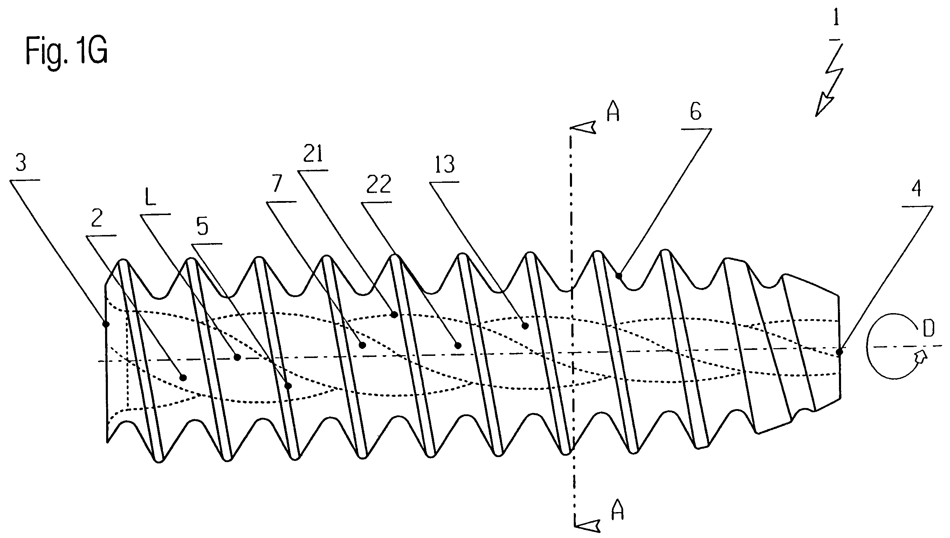

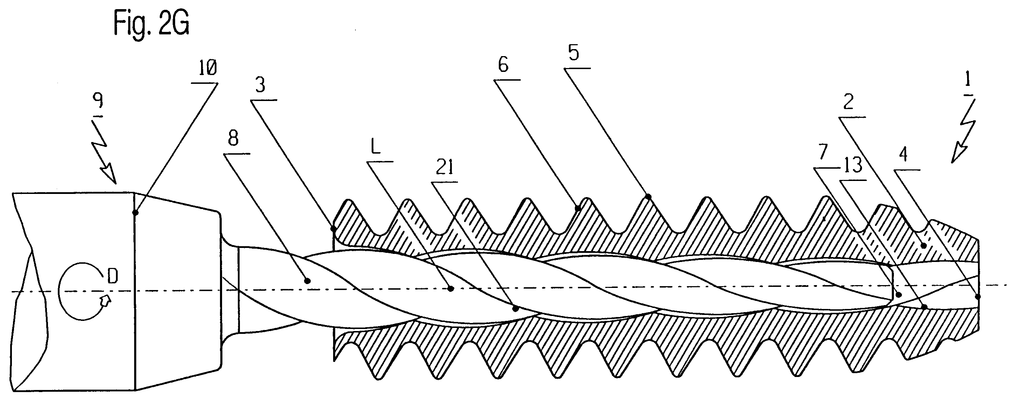

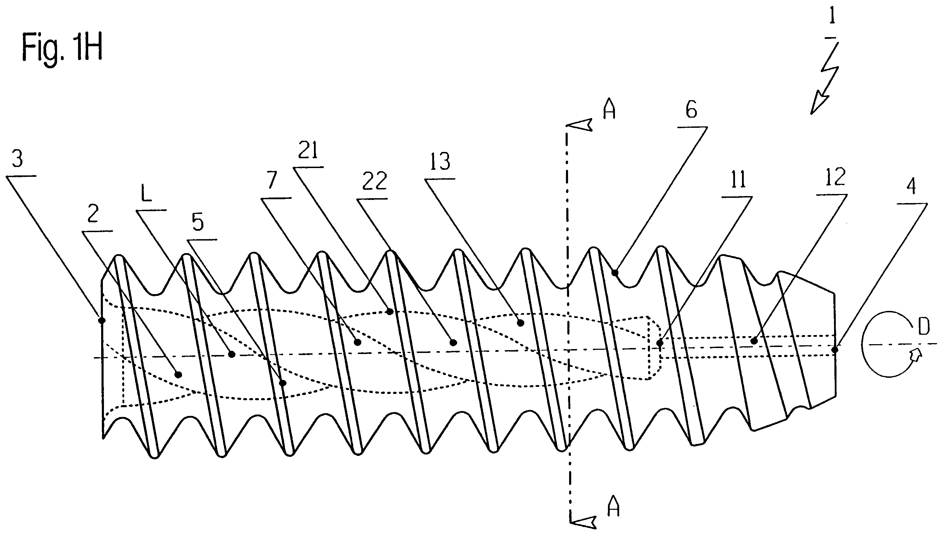

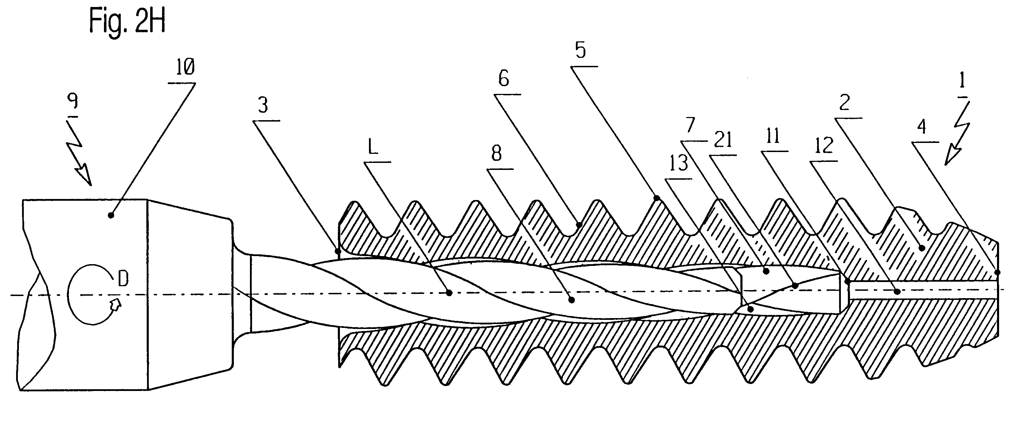

Im Schraubenkörper 2 ist ein zur Längsachse L koaxialer Kanal 7 vorgesehen. Dieser Kanal 7 erstreckt sich bis in den Bereich des distalen Endes 4, in den Ausbildungen nach Fig. 1A, 1C, 1E und 1G sowie 2A, 2C, 2E und 2G ganz bis zum distalen Ende 4, in den Ausbildungen nach Fig. 1B, 1D, 1F und 1H sowie 2B, 2D, 2F und 2H nur bis in die Nähe des distalen Endes 4. Im letztgenannten Fall erstreckt sich zwischen einem Ende 11 des Kanals 7 und dem distalen Ende 4 ein zur Längsachse L koaxialer kreiszylindrischer Hilfskanal 12, dessen Rolle weiter unten erläutert wird.

Am proximalen Ende 3 ist der Kanal 7 gegen aussen offen, um einen Schaft 8 eines Schraubendrehers 9 aufnehmen zu können. In Fig. 2A bis 2H ist der Schaft 8 in Seitenansicht im Kanal 7 des Schraubenkörpers 2 koaxial eingesetzt dargestellt, während der Schraubendreher 9 mit einem koaxialen Griff 10 versehen ist, der gebrochen dargestellt ist. Der Schaft 8 hat eine zum Kanal 7 komplementär passende Form, die im nachstehenden näher erläutert wird. Mit Hilfe des Griffs 10 ist der der Schaft 8 des Schraubendrehers 9 in den Kanal 7 einführbar und daraus entfernbar und somit der Schraubendreher 9 (bei in den Kanal 7 eingeführtem Schaft 8) zum Drehen der Schraube 1 betätigbar.

Der Hilfskanal 12 nach Fig. 1B, 1D, 1F und 1H sowie 2B, 2D, 2F und 2H vermeidet, dass der Schaft 8 bei seiner Einführung in den Kanal 7 als Kolben wirkt und Luft im Kanal 7 komprimiert. So braucht bei der Einführung des Schafts 8 in den Kanal 7 keine Luft in einem Spalt zwischen dem Schaft 8 und dem Kanal 7 zu entweichen, was ermöglicht, zwischen dem Schaft 8 und dem Kanal 7 bzw. einer Kanalwand 13 des Kanals 7 ein nur sehr kleines Spiel zu lassen und folglich eine optimale Übertragung der Kraft vom Schraubendreher 9 auf die Schraube 1 zu gewährleisten. Zudem kann der Hilfskanal 12 auf beispielsweise aus US-A-4950270 bekannte Weise als Durchgang für einen Führungsstift oder einen Führungsdraht dienen, wenn der Schaft 8 des Schraubendrehers 9 entsprechend kanüliert ist.

In den Ausbildungen nach Fig. 1A und 2A sowie 1B und 2B weisen der Kanal 7 und der Schaft 8 geradlinige Mantellinien 18 auf, die parallel zur Längsachse L sind. Der Kanal 7 und der Schaft 8 sind also in ein zylindrisches umhüllendes Volumen einschreibbar.

In den Ausbildungen nach Fig. 1C und 2C sowie 1D und 2D weisen der Kanal 7 und der Schaft 8 geradlinige Mantellinie 19 auf, die schräg d.h. im Winkel zur Längsachse L mit einem halben Öffnungswinkel von 2° liegen. Der Kanal 7 und der Schaft 8 sind also in ein kegelstumpfförmiges umhüllendes Volumen mit einem halben Öffnungswinkel von 2° einschreibbar.

In den Ausbildungen nach Fig. 1E und 2E sowie 1F und 2F weisen der Kanal 7 und der Schaft 8 schraubenförmige Mantellinien 20 auf, die jeweils windschief zur Längsachse L auf einem zur Längsachse L koaxialen Zylinder liegen. Der Kanal 7 und der Schaft 8 sind also in ein zylindrisches umhüllendes Volumen einschreibbar.

In den Ausbildungen nach Fig. 1G und 2G sowie 1H und 2H weisen der Kanal 7 und der Schaft 8 schraubenförmige Mantellinien 21 auf, die jeweils windschief zur Längsachse L auf einem zur Längsachse L koaxialen Kegelstumpf mit einem halben Öffnungswinkel von 2° liegen. Der Kanal 7 und der Schaft 8 sind also in ein kegelstumpfförmiges umhüllendes Volumen mit einem halben Öffnungswinkel von 2° einschreibbar.

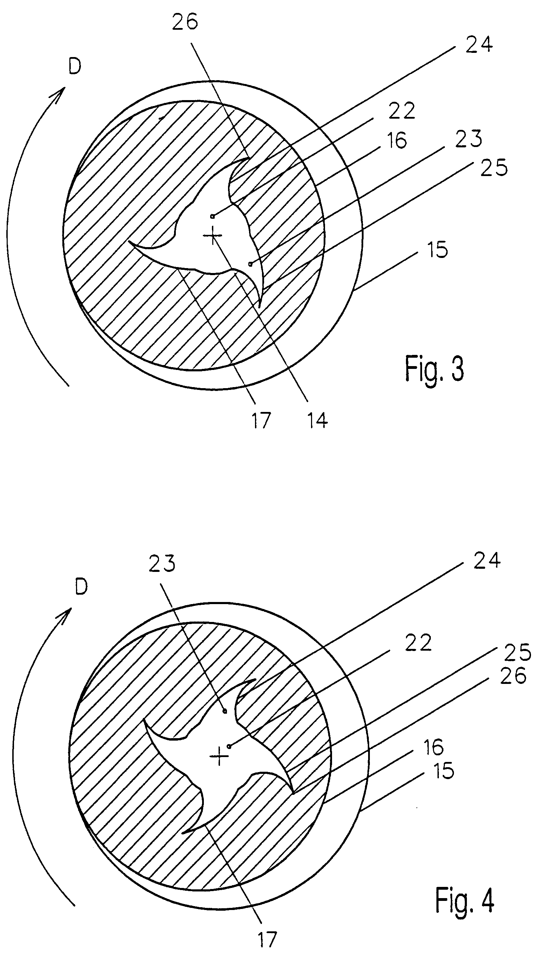

Fig. 3 bis 10 zeigen Ausbildungen der Kanalwand 13 als Spur davon in der Ebene eines jeweiligen Querschnitts des Schraubenkörpers 2 rechtwinklig zur Längsachse L. Im jeweiligen Querschnitt bzw. in dessen Ebene entspricht der Längsachse L eine Spur 14, der Aussenfläche 5 des Schraubenkörpers 2 eine Spur 15, dem Aussengewinde 6 eine Spur 16, und der Kanalwand 13 eine Spur 17.

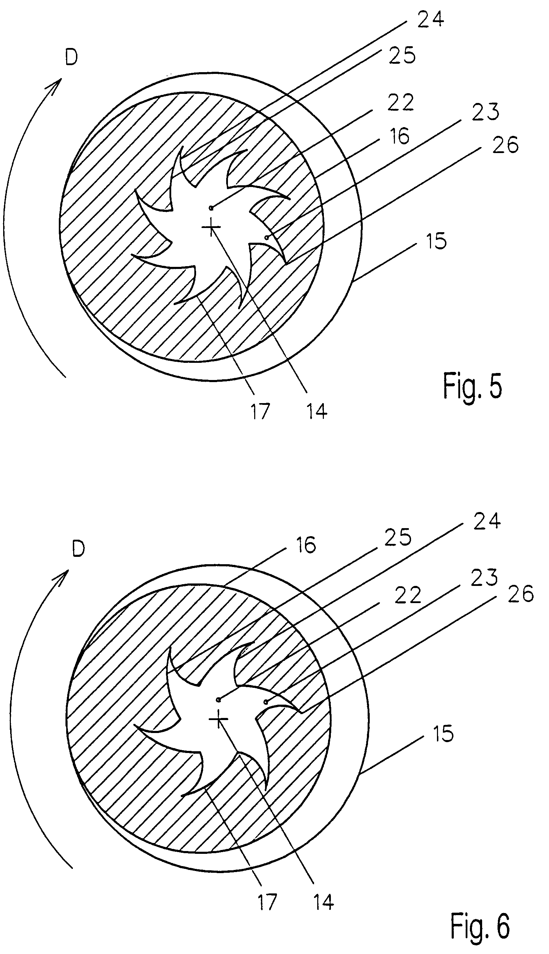

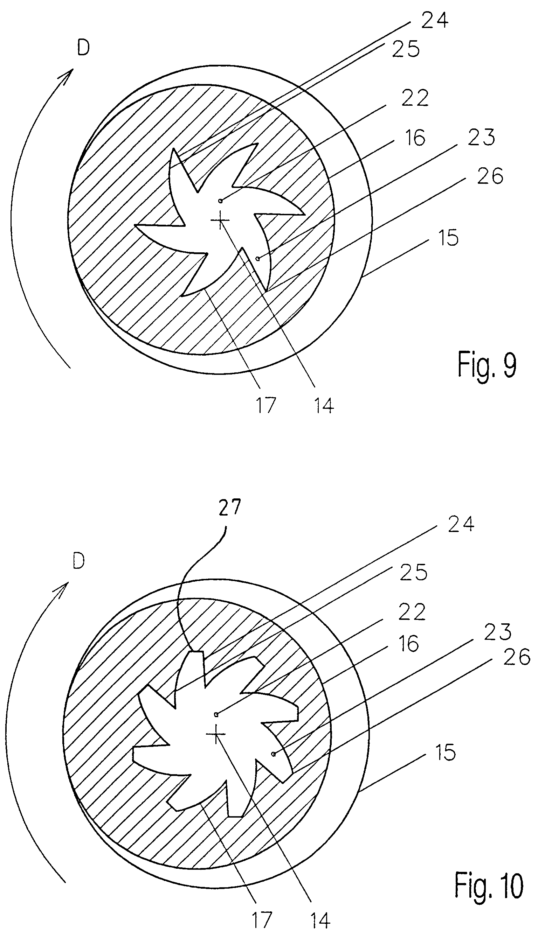

Diese Spur 17 der Kanalwand 13 ist eine geschlossene Umrisslinie, die in diskreten regelmässigen Drehschritten um ein von der Spur 14 der Längsachse L gebildetes Symmetriezentrum radialsymmetrisch ist, nämlich in der Ausbildung gemäss Fig. 3 in drei Drehschritten von 120°, in der Ausbildung gemäss Fig. 4 in vier Drehschritten von 90°, in den Ausbildungen gemäss Fig. 5 und 10 in acht Drehschritten von 45°, und in den Ausbildungen gemäss Fig. 6 bis 9 jeweils in sechs Drehschritten von 60°. Aus der Form der Spur 17 der Kanalwand 13 ergibt sich, dass die Kanalwand 13 im Kanal 7 einen axialen Kanalbereich 22 und eine Mehrzahl von um den Kanalbereich 22 regelmässig angeordneten Lappenbereichen 23 definiert, deren Anzahl in der Ausbildung gemäss Fig. 3 gleich drei, in der Ausbildung gemäss Fig. 4 gleich vier, in den Ausbildungen gemäss Fig. 5 und 10 gleich acht und in den Ausbildungen gemäss Fig. 6 bis 9 jeweils gleich sechs ist. Somit besteht der Kanal 7 im wesentlichen aus der Vereinigung des axialen Kanalbereiches 22 und der jeweiligen Lappenbereiche 23.

In jedem Lappenbereich 23 weist die Kanalwand 13 ein Paar von Flanken auf, die sich im wesentlichen vom Kanalbereich 22 ausgehend radial nach aussen erstrecken. Mit Bezug auf die Drehrichtung D werden in diesem Paar von Flanken eine vorlaufende Flanke und eine nachlaufende Flanke 25 definiert. In den Ausbildungen gemäss Fig. 3 bis 12 entspricht der vorlaufenden Flanke eine Spur 24 und der nachlaufenden Flanke eine Spur 25.

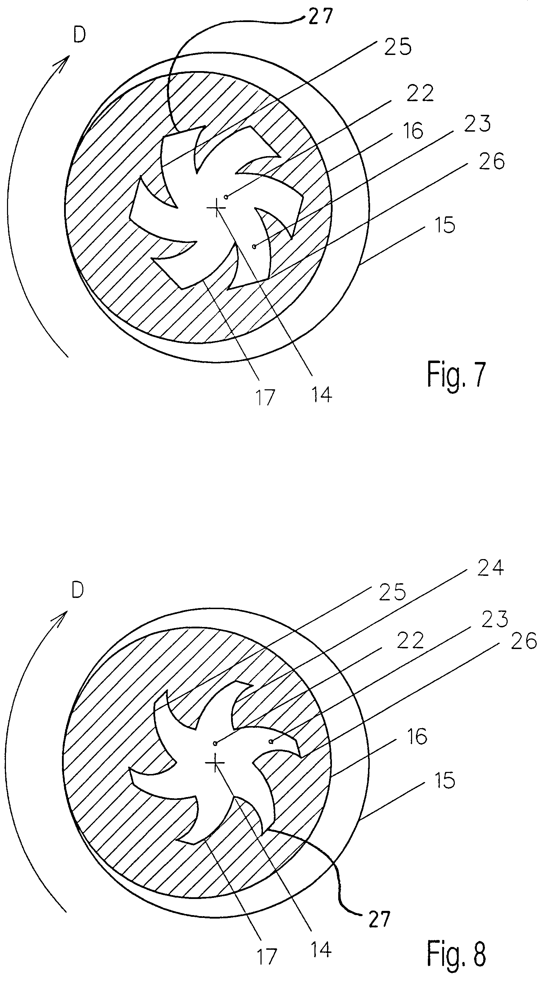

In den Ausbildungen gemäss Fig. 3 bis 6 sowie gemäss Fig. 9 erstrecken sich die Spuren 24 und 25 der beiden Flanken jeweils bis zu einer Spur 26 eines dem Kanalbereich 22 abgewandten Endes des Lappenbereiches 23, so dass im Raume gesehen die beiden Flanken am Ende des Lappenbereiches 23 zu einer Kante zusammenlaufen. In den Ausbildungen gemäss Fig. 7, 8 und 10 erstrecken sich die Spuren 24 und 25 der beiden Flanken jeweils bis in Nähe der Spur des dem Kanalbereich 22 abgewandten Endes 26 des Lappenbereiches 23, jedoch erstreckt sich nur eine dieser Flanken, nämlich in Fig. 7 und 8 die vorlaufende Flanke mit der Spur 24 und in Fig. 10 die nachlaufende Flanke mit der Spur 25, ganz bis zur Spur 26 des Endes des Lappenbereiches 23, so dass im Raume gesehen der Lappenbereich 23 stumpf endet und im Bereich seines Endes eine Abschlusswandung mit einer Spur 27 aufweist.

Beachtenswert ist dabei, dass in jedem Lappenbereich 23 zumindest die vorlaufende Flanke mit der Spur 24 zumindest bereichsweise eine mit wachsendem radialem Abstand von der Spur 14 der Längsachse L in der Drehrichtung D zunehmend vorlaufende Form aufweist. In den Ausbildungen gemäss Fig. 7, 9 und 10 haben beide Flanken mit den Spuren 24 und 25 eine Form, die über ihre ganze radiale Länge mit wachsendem radialem Abstand in der Drehrichtung D zunehmend voreilt. In den Ausbildungen gemäss Fig. 3 bis 6 sowie gemäss Fig. 8 hat die vorlaufende Flanke mit der Spur 24 eine Form, die etwa über einer radial äusseren Hälfte ihrer radialen Länge (also in Nähe der Spur 26 des Endes des Lappenbereiches 23) mit wachsendem radialem Abstand in der Drehrichtung D zunehmend voreilt. In den Ausbildungen gemäss Fig. 3 bis 10 hat die nachlaufende Flanke mit der Spur 25 stets eine Form, die über ihre ganze radiale Länge mit wachsendem radialem Abstand in der Drehrichtung D zunehmend voreilt, jedoch ist auch die Abschlusswandung mit der Spur 27 zu berücksichtigen, welche im genannten Sinne bei der Ausbildung gemäss Fig. 8 voreilt und bei den Ausbildungen gemäss Fig. 7 und 10 nicht voreilt.

Es wird hier noch klargestellt, was unter "Voreilen einer Fläche in einer vorgegebenen Drehrichtung mit wachsendem radialem Abstand von einer Achse" zu verstehen ist. In einem Querschnitt der Fläche rechtwinklig zur Achse werden zwei Laufpunkte einer Spur der Fläche betrachtet. Ausgehend von der Achse ist ein erster Radius auf den zur Achse näheren Laufpunkt und ein zweiter Radius auf den von der Achse entfernteren Laufpunkt gerichtet. Um den ersten Radius mit dem zweiten Radius in Kongruenz zu bringen, wird eine Drehung benötigt. Erfolgt diese Drehung in der vorgegebenen Drehrichtung, so ist die Spur bzw. die Fläche in der vorgegebenen Drehrichtung "voreilend", weil in der vorgegebenen Drehrichtung und von der Achse aus betrachtet der entferntere Laufpunkt "vor" dem näheren Laufpunkt liegt.

In den Ausbildungen gemäss Fig. 3 bis 6 sowie gemäss Fig. 8 bis 10 verjüngt sich jeder Lappenbereich 23 zu seinem Ende mit der Spur 26 hin. In anderen Worten, in diesen Ausbildungen sind die beiden Flanken mit den Spuren 24 und 25 in einem Abstand voneinander, der sich mit wachsendem radialem Abstand von der Längsachse L zur Spur 26 des Endes des Lappenbereiches 23 hin verjüngt. Diese Form des Lappenbereiches 23 kommt dadurch zustande, dass zumindest in Nähe des Endes des Lappenbereiches 23 mit der Spur 26 die vorlaufende Flanke mit der Spur 24 zur Drehrichtung D hin konkav und die nachlaufende Flanke mit der Spur 25 entgegengesetzt zur Drehrichtung D konvex gekrümmt ist. Wenn die beiden Flanken mit den Spuren 24 und 25 am Ende des Lappenbereiches 23 mit der Spur 26 zu einer Kante zusammenlaufen, ergibt sich daraus eine Umrisslinie der Spur 17 der Kanalwand 13 mit einem sternförmigen Linienzug, der um die Spur 14 der Längsachse L radialsymmetrisch ist und zur Drehrichtung D hin vorgezogene sichelförmige Zacken in entsprechender Anzahl aufweist. Im gegenteiligen Fall, wenn die beiden Flanken mit den Spuren 24 und 25 am Ende des Lappenbereiches 23 mit der Spur 26 nicht zu einer Kante zusammenlaufen, ergibt sich daraus eine Umrisslinie der Spur 17 der Kanalwand 13 mit einem schaufelradförmigen Linienzug, der um die Spur 14 der Längsachse L radialsymmetrisch ist und zur Drehrichtung D hin vorgezogene etwa sichelförmige Lappen in entsprechender Anzahl aufweist.

In der Ausbildung gemäss Fig. 7 bleibt hingegen jeder Lappenbereich 23 etwa gleich breit, weil darin die beiden Flanken mit den Spuren 24 und 25 in etwa gleichbleibendem Abstand voneinander sind. Es ergibt sich daraus eine Umrisslinie der Spur 17 der Kanalwand 13 mit einem schaufelradförmigen Linienzug, der um die Spur 14 der Längsachse L radialsymmetrisch ist und zur Drehrichtung D hin vorgezogene, etwa ringsegmentförmige Lappen von etwa gleichbleibender Breite in entsprechender Anzahl aufweist.

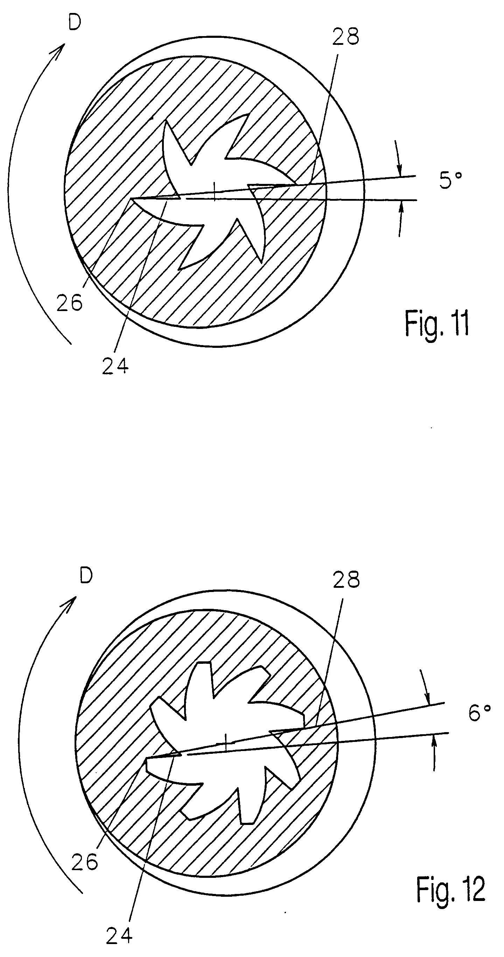

In den Ausbildungen gemäss Fig. 9 und 10 ist in jedem Lappenbereich 23 die Spur 24 der vorlaufenden Flanke im wesentlichen geradlinig und zur genannten Drehrichtung hin geneigt. Diese Neigung der vorlaufenden Flanke 24 beträgt bis zu 10° und vorzugsweise etwa 5°. In Fig. 11 ist die Ausbildung gemäss Fig. 9 und in Fig. 12 die Ausbildung gemäss Fig. 10 identisch dargestellt, jedoch aus Gründen der besseren Lesbarkeit ohne Schraffur und mit nur einigen der Bezugszeichen, hingegen mit hinzugefügter Bemessung eines Winkels zwischen der Spur 24 der vorlaufenden Flanke und der Spur 28 einer durch das Ende bzw. durch dessen Spur 26 gehenden diametralen Ebene. Der wert dieses Winkels ist beispielsweise in der Ausbildung gemäss Fig. 9 und 11 gleich etwa 5° und in der Ausbildung gemäss Fig. 10 und 12 gleich etwa 6°.

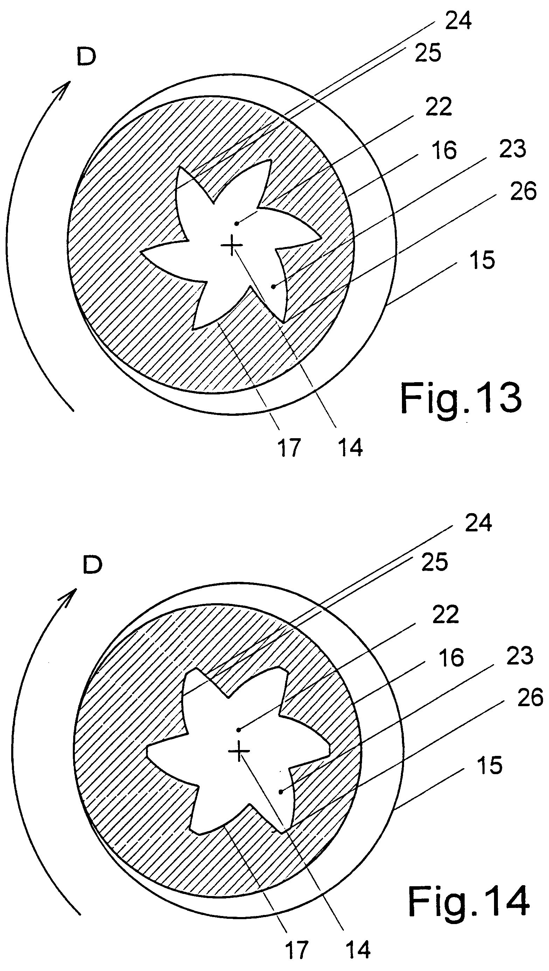

Fig. 13 bis 16 zeigen Ausbildungen der Kanalwand 13 als Spur davon in der Ebene eines jeweiligen Querschnitts des Schraubenkörpers 2 rechtwinklig zur Längsachse L. Im jeweiligen Querschnitt bzw. in dessen Ebene entspricht der Längsachse L eine Spur 14, der Aussenfläche 5 des Schraubenkörpers 2 eine Spur 15, dem Aussengewinde 6 eine Spur 16, und der Kanalwand 13 eine Spur 17.

Diese Spur 17 der Kanalwand 13 ist eine geschlossene Umrisslinie, die in diskreten regelmässigen Drehschritten um ein von der Spur 14 der Längsachse L gebildetes Symmetriezentrum radialsymmetrisch ist, beispielsweise in den gezeigten Ausbildungen gemäss Fig. 13 bis 16 jeweils in sechs Drehschritten von 60°, was aber andere, nicht dargestellte Ausbildungen beispielsweise in drei Drehschritten von 120°, in vier Drehschritten von 90°, in acht Drehschritten von 45° usw. keineswegs ausschliesst. Aus der Form der Spur 17 der Kanalwand 13 ergibt sich, dass die Kanalwand 13 im Kanal 7 einen axialen Kanalbereich 22 und eine Mehrzahl von um den Kanalbereich 22 regelmässig angeordneten Lappenbereichen 23 definiert, deren Anzahl in den gezeigten Ausbildungen gemäss Fig. 13 bis 16 jeweils gleich sechs ist, aber in anderen, nicht dargestellten Ausbildungen gleich drei, vier, acht usw. sein kann. Somit besteht der Kanal 7 im wesentlichen aus der Vereinigung des axialen Kanalbereiches 22 und der jeweiligen Lappenbereiche 23.

In jedem Lappenbereich 23 weist die Kanalwand 13 ein Paar von Flanken auf, die sich im wesentlichen vom Kanalbereich 22 ausgehend radial nach aussen erstrecken. Mit Bezug auf die Drehrichtung D werden in diesem Paar von Flanken eine vorlaufende Flanke und eine nachlaufende Flanke 25 definiert. In den Ausbildungen gemäss Fig. 13 bis 16 entspricht der vorlaufenden Flanke eine Spur 24 und der nachlaufenden Flanke eine Spur 25.

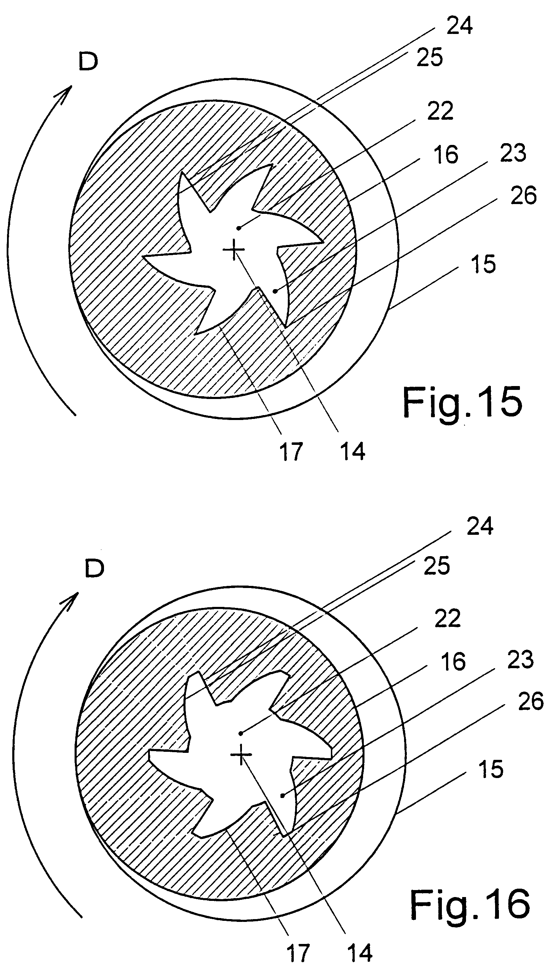

In den Ausbildungen gemäss Fig. 13 und 15 erstrecken sich die Spuren 24 und 25 der beiden Flanken jeweils bis zu einer Spur 26 eines dem Kanalbereich 22 abgewandten Endes des Lappenbereiches 23, so dass im Raume gesehen die beiden Flanken am Ende des Lappenbereiches 23 zu einer Kante zusammenlaufen. In den Ausbildungen gemäss Fig. 14 und 16 erstrecken sich die Spuren 24 und 25 der beiden Flanken jeweils bis in Nähe der Spur des dem Kanalbereich 22 abgewandten Endes 26 des Lappenbereiches 23, jedoch erstreckt sich nur eine dieser Flanken, nämlich die nachlaufende Flanke mit der Spur 25, ganz bis zur Spur 26 des Endes des Lappenbereiches 23, so dass im Raume gesehen der Lappenbereich 23 stumpf endet und im Bereich seines Endes eine Abschlusswandung mit einer Spur 27 aufweist.

Beachtenswert ist dabei, dass im dargestellten Querschnitt des Schraubenkörpers 2 rechtwinklig zur Längsachse L betrachtet, jeder Lappenbereich 23 in bezug auf eine durch die Längsachse L gehende diametrale Ebene asymmetrisch ausgebildet ist. Um jedem Lappenbereich 23 ein sägezahnähnliches Profil mit (beim Verlauf des radialen Abstands von der Längsachse L entlang der Umrisslinie 17) durchschnittlich steilerem Anstieg der vorlaufenden Flanke 24 und flacherem Abfall der nachlaufenden Flanke 25 zu geben, ist wie dargestellt jeweils ein der vorlaufenden Flanke 24 entsprechender Abschnitt der Umrisslinie 17 kürzer ausgebildet als ein der nachlaufenden Flanke 25 entsprechender Abschnitt der Umrisslinie 17.

In den gezeigten Ausbildungen gemäss Fig. 13 bis 16 verjüngt sich jeder Lappenbereich 23 zu seinem Ende mit der Spur 26 hin. In anderen Worten, die beiden Flanken mit den Spuren 24 und 25 sind in einem Abstand voneinander, der sich mit wachsendem radialem Abstand von der Längsachse L zur Spur 26 des Endes des Lapperbereiches 23 hin verjüngt. Diese Form des Lappenbereiches 23 kommt dadurch zustande, dass zumindest in Nähe des Endes des Lappenbereiches 23 mit der Spur 26 die vorlaufende Flanke mit der Spur 24 zur Drehrichtung D hin weniger konvex d.h. durchschnittlich mit grösserem Krümmungsradius gekrümmt ist, als die nachlaufende Flanke mit der Spur 25 entgegengesetzt zur Drehrichtung D konvex gekrümmt ist. In diesem Zusammenhang ist eine ebene Ausbildung der vorlaufenden Flanke mit der Spur 24 als Grenzfall einer konvexen Spur mit unendlich grossem Krümmungsradius zu verstehen. Wenn die beiden Flanken mit den Spuren 24 und 25 am Ende des Lappenbereiches 23 mit der Spur 26 zu einer Kante zusammenlaufen, ergibt sich daraus eine Umrisslinie der Spur 17 der Kanalwand 13 mit einem spitzen, sternförmigen Linienzug, der um die Spur 14 der Längsachse L radialsymmetrisch ist und zur Drehrichtung D hin vorgezogene Zacken in entsprechender Anzahl aufweist. Im gegenteiligen Fall, wenn die beiden Flanken mit den Spuren 24 und 25 am Ende des Lappenbereiches 23 mit der Spur 26 nicht zu einer Kante zusammenlaufen, ergibt sich daraus eine Umrisslinie der Spur 17 der Kanalwand 13 mit einem stumpfen Linienzug, der um die Spur 14 der Längsachse L radialsymmetrisch ist und zur Drehrichtung D hin vorgezogene Lappen in entsprechender Anzahl aufweist.

Hat der Kanal 7, wie in den Ausbildungen nach Fig. 1A und 2A sowie 1B und 2B, geradlinige und zur Längsachse L parallele Mantellinien 18, so liegt die vorlaufende Flanke im wesentlichen in einer Ebene, und die Umrisslinien bzw. Spuren 17 der Kanalwand 13 in zwei voneinander verschiedenen Querschnitte des Schraubenkörpers 2 rechtwinklig zur Längsachse L sind miteinander kongruent.

Hat der Kanal 7, wie in den Ausbildungen nach Fig. 1C und 2C sowie 1D und 2D, geradlinige und zur Längsachse L schräg liegende Mantellinien 19, so ist die vorlaufende Flanke in Richtung vom proximalen Ende 3 zum distalen Ende 4 hin verjüngt, und die Umrisslinien bzw. Spuren 17 der Kanalwand 13 in zwei voneinander verschiedenen Querschnitte des Schraubenkörpers 2 rechtwinklig zur Längsachse L sind Ähnlichkeitsabbildungen voneinander, die durch zentrische Streckung um die Spur 14 der Längsachse L zur Kongruenz bringbar sind.

Hat der Kanal 7, wie in den Ausbildungen nach Fig. 1E und 2E sowie 1F und 2F, schraubenförmig um die Längsachse L zur Drehrichtung D hin verdrillte Mantellinien 20, so ist auch die vorlaufende Flanke schraubenförmig um die Längsachse L zur Drehrichtung D hin verdrillt, und die Umrisslinien bzw. Spuren 17 der Kanalwand 13 in zwei voneinander verschiedenen Querschnitte des Schraubenkörpers 2 rechtwinklig zur Längsachse L sind Kongruenzabbildungen voneinander, die durch reine Drehung um die Spur 14 der Längsachse L zur Kongruenz bringbar sind.

Hat schliesslich der Kanal 7, wie in den Ausbildungen nach Fig. 1G und 2G sowie 1H und 2H, schraubenförmig um die Längsachse L zur Drehrichtung D hin verdrillte und gleichzeitig windschief zur Längsachse L liegende Mantellinien 21, so ist auch die vorlaufende Flanke sowohl schraubenförmig um die Längsachse L zur Drehrichtung D hin verdrillt als auch in Richtung vom proximalen Ende 3 zum distalen Ende 4 hin verjüngt, und die Umrisslinien bzw. Spuren 17 der Kanalwand 13 in zwei voneinander verschiedenen Querschnitte des Schraubenkörpers 2 rechtwinklig zur Längsachse L sind Ähnlichkeitsabbildungen voneinander, die durch eine Kombination von reiner Drehung und zentrischer Streckung um die Spur 14 der Längsachse L zur Kongruenz bringbar sind.

Im Falle der Verdrillung beträgt der Schraubengang von beispielsweise einer Umdrehung in 10 cm. Im Falle der Verjüngung beträgt der halbe Öffnungswinkel bis zu 10° und vorzugsweise etwa 2°.

Da der Schaft 8 des Schraubendrehers 9 zum Eindrehen einer Schraube 1 in Knochenmaterial eines Patienten während eines osteochirurgischen Eingriffs in den Kanal 7 einführbar und daraus entfernbar sein muss, weist der Schaft 8 eine zum Kanal 7 komplementär passende Form auf, die den vorangehend beschriebenen Ausbildungen der Schraube entspricht und bei im Querschnitt entsprechendem Umriss zylindrisch oder kegelstumpfförmig sowie geradlinig oder verdrillt sein kann.

Die beschriebenen Ausbildungen der Schraube und des entsprechenden Schafts des Schraubendrehers mit jeweils 3 bis 8 Zacken bzw. Lappen sind zwar bevorzugt, wobei Ausbildungen mit 6 Zacken bzw. Lappen besonders bevorzugt sind. Es ist jedoch zu verstehen, dass die Erfindung auch Ausbildungen der Schraube und des entsprechenden Schafts des Schraubendrehers mit nur 2 oder mit mehr als 8 Zacken bzw. Lappen, beispielsweise mit 9, 10 oder mehr Zacken bzw. Lappen umfasst.

## Patentansprüche

1. Schraube (1) aus bioabbaubarem Material für osteochirurgische Zwecke mit einem länglichen Schraubenkörper (2), der sich in Richtung einer Längsachse (L) davon zwischen einem proximalen (3) und einem distalen (4) Ende davon erstreckt, wobei
eine Aussenfläche (5) des Schraubenkörpers (2) mit einem zur Längsachse (L) koaxialen Aussengewinde (6) versehen ist, das zum Führen und Halten der Schraube (1) an Knochenmaterial eines Patienten bestimmt ist und eine Drehrichtung (D) um die Längsachse (L) für das Einschrauben des Schraubenkörpers (2) und das Vorrücken des distalen Endes (4) in das Knochenmaterial definiert,
im Schraubenkörper (2) ein länglicher, zur Längsachse (L) koaxialer Kanal (7) vorgesehen ist, der am proximalen Ende (3) zur Aufnahme eines Schafts (8) eines zum Drehen der Schraube (1) bestimmten Schraubendrehers (9) offen ist,
der Kanal (7) eine Kanalwand (13) aufweist, deren Form um die Längsachse (L) in diskreten regelmässigen Drehschritten radialsymmetrisch ist und in einem Querschnitt des Schraubenkörpers (2) rechtwinklig zur Längsachse (L) eine Spur (17) ergibt, welche eine um eine Spur (14) der Längsachse (L) in diskreten regelmässigen Drehschritten radialsymmetrische geschlossene Umrisslinie (17) ist,
die Kanalwand (13) einen axialen Kanalbereich (22) und eine Mehrzahl von um den Kanalbereich (22) regelmässig angeordneten Lappenbereichen (23) definiert, so dass der Kanal (7) im wesentlichen aus der Vereinigung des axialen Kanalbereiches (22) und der Lappenbereiche (23) besteht, und
in jedem Lappenbereich die Kanalwand (13) ein Paar von Flanken (24,25) aufweist, die sich im wesentlichen vom Kanalbereich (22) bis in Nähe eines dem Kanalbereich (22) abgewandten Endes (26) des Lappenbereiches (23) erstrecken und unter denen mit Bezug auf die genannte Drehrichtung (D) jeweils eine vorlaufende Flanke (24) und eine nachlaufende Flanke (25) definiert ist,
**dadurch gekennzeichnet, dass** der Kanal (7) sich bis in den Bereich des distalen Endes (4) der Schraube (1) erstreckt und, in einem Querschnitt des Schraubenkörpers (2) rechtwinklig zur Längsachse (L) betrachtet, jeder Lappenbereich (23) in bezug auf eine durch die Längsachse (L) gehende diametrale Ebene asymmetrisch ausgebildet ist, wobei ein der vorlaufenden Flanke (24) entsprechender Abschnitt der Umrisslinie (17) kürzer ist als ein der nachlaufenden Flanke (25) entsprechender Abschnitt der Umrisslinie (17), so dass der Lappenbereich (23) beim Verlauf des radialen Abstands von der Längsachse (L) entlang der Umrisslinie (17) ein sägezahnähnliches Profil mit durchschnittlich steilerem Anstieg der vorlaufenden Flanke (24) und flacherem Abfall der nachlaufenden Flanke (25) aufweist.

2. Schraube nach Anspruch 1, **dadurch gekennzeichnet, dass** der Kanal (7) sich bis in den Bereich des distalen Endes (4) der Schraube (1) erstreckt und in jedem Lappenbereich (23) mindestens eine der genannten Flanken (24) zumindest in Nähe des genannten Endes (26) des Lappenbereiches (23) eine mit wachsendem radialem Abstand von der Längsachse (L) in der genannten Drehrichtung (D) zunehmend vorlaufende Form aufweist.

3. Schraube nach Anspruch 1 oder 2, **dadurch gekennzeichnet, dass** die Flanken (24,25) schraubenförmig um die Längsachse (L) zur genannten Drehrichtung (D) hin verdrillt sind, derart, dass in zwei voneinander verschiedenen Querschnitten des Schraubenkörpers (2) rechtwinklig zur Längsachse (L) die Umrisslinien (17) Kongruenzabbildungen voneinander darstellen, die durch reine Drehung um die Spur der Längsachse (L) zur Kongruenz bringbar sind.

4. Schraube nach Anspruch 1 oder 2, **dadurch gekennzeichnet, dass** die Flanken (24,25) in Richtung vom proximalen (3) zum distalen (4) Ende hin verjüngt sind, derart, dass in zwei voneinander verschiedenen Querschnitten des Schraubenkörpers (2) rechtwinklig zur Längsachse (L) die Umrisslinien (17) Ähnlichkeitsabbildungen voneinander darstellen, die durch zentrische Streckung um die Spur (14) der Längsachse (L) zur Kongruenz bringbar sind.

5. Schraube nach den Ansprüchen 3 und 4, **dadurch gekennzeichnet, dass** die Flanken (24,25) sowohl verdrillt als auch verjüngt sind, derart, dass in zwei voneinander verschiedenen Querschnitten des Schraubenkörpers (2) rechtwinklig zur Längsachse (L) die Umrisslinien (17) Ähnlichkeitsabbildungen voneinander darstellen, die durch eine Kombination von reiner Drehung und zentrischer Streckung um die Spur (14) der Längsachse (L) zur Kongruenz bringbar sind.

6. Schraube nach einem der Ansprüche 4 und 5, **dadurch gekennzeichnet, dass** die Verjüngung in Richtung vom proximalen (3) zum distalen (4) Ende hin einem halben Öffnungswinkel von bis zu 10° und vorzugsweise von etwa 2° entspricht.

7. Schraube nach Anspruch 1 oder 2, **dadurch gekennzeichnet, dass** in jedem Lappenbereich (23) die beiden Flanken (24,25) in einem Abstand voneinander verlaufen, der sich mit wachsendem radialem Abstand von der Längsachse (L) zum Ende (26) des Lappenbereiches (23) hin verjüngt.

8. Schraube nach Anspruch 7, **dadurch gekennzeichnet, dass** die beiden Flanken (24,25) am Ende (26) des Lappenbereiches 23) zu einer Kante zusammenlaufen.

9. Schraube nach Anspruch 1 oder 2, **dadurch gekennzeichnet, dass** in jedem Lappenbereich (23) die nachlaufende Flanke (25) zumindest in Nähe des Endes (26) des Lappenbereiches (23) entgegengesetzt zur genannten Drehrichtung (D) konvex gekrümmt ist.

10. Schraube nach Anspruch 1, **dadurch gekennzeichnet, dass** in jedem Lappenbereich (23) die vorlaufende Flanke (24) zumindest in Nähe des Endes (26) des Lappenbereiches (23) zur genannten Drehrichtung (D) hin konvex gekrümmt ist oder im wesentlichen in einer durch die Längsachse (L) gehende diametrale Ebene liegt.

11. Schraube nach Anspruch 2, **dadurch gekennzeichnet, dass** in jedem Lappenbereich (23) die vorlaufende Flanke (24) zumindest in Nähe des Endes (26) des Lappenbereiches (23) zur genannten Drehrichtung (D) hin konkav gekrümmt ist.

12. Schraube nach nach Anspruch 1 oder 2, **dadurch gekennzeichnet, dass** in einem Querschnitt des Schraubenkörpers (2) rechtwinklig zur Längsachse (L) die Umrisslinie (17) im wesentlichen einem um die Spur (14) der Längsachse (L) radialsymmetrischen sternförmigen Linienzug mit 3 bis 8 und vorzugsweise 6 zur genannten Drehrichtung (D) hin vorgezogenen Zacken oder Lappen (23) entspricht.

13. Schraube nach den Ansprüchen 2 und 12, **dadurch gekennzeichnet, dass** die Zacken oder Lappen (23) etwa sichelförmig sind.

14. Schraube nach Anspruch 2, **dadurch gekennzeichnet, dass** in jedem Lappenbereich (23) die beiden Flanken (24,25) in etwa gleichbleibendem Abstand voneinander verlaufen.

15. Schraube nach Anspruch 14, **dadurch gekennzeichnet, dass** in einem Querschnitt des Schraubenkörpers (2) rechtwinklig zur Längsachse (L) die Umrisslinie (17) im wesentlichen einem um die Spur (14) der Längsachse (L) radialsymmetrischen schaufelradförmigen Linienzug mit 3 bis 8 und vorzugsweise 6 zur genannten Drehrichtung (D) hin vorgezogenen, etwa ringsegmentförmigen Lappen (23) von etwa gleichbleibender Breite entspricht.

16. Schraube nach Anspruch 2, **dadurch gekennzeichnet, dass** in jedem Lappenbereich (23) in einem Querschnitt des Schraubenkörpers (2) rechtwinklig zur Längsachse (L) eine Spur (24) der vorlaufenden Flanke (24) im wesentlichen geradlinig und zur genannten Drehrichtung (D) hin geneigt ist.

17. Schraube nach Anspruch 16, **dadurch gekennzeichnet, dass** die Neigung der vorlaufenden Flanke (24) bis zu 10° und vorzugsweise etwa 5° beträgt.

18. Schraubendreher (9) zum Eindrehen einer Schraube (1) nach Anspruch 1 in Knochenmaterial eines Patienten während eines osteochirurgischen Eingriffs, wobei der Schraubendreher (9) mit einem Griff (10) und einem daran befestigten länglichen Schaft (8) versehen ist, der in einen Kanal (7) der Schraube (1) einführbar und daraus entfernbar ist, wobei um eine Längsachse (L) des Schaftes (8) eine Drehrichtung (D) für das Einschrauben der Schraube (1) in das Knochenmaterial definiert ist, und wobei
der Schaft (8) eine Schaftmantelfläche aufweist, deren Form um die Längsachse (L) in diskreten regelmässigen Drehschritten radialsymmetrisch ist und in einem Querschnitt des Schaftes (8) rechtwinklig zur Längsachse (L) eine Spur (17) ergibt, welche eine um eine Spur (14) der Längsachse (L) in diskreten regelmässigen Drehschritten radialsymmetrische geschlossene Umrisslinie (17) ist,
die Schaftmantelfläche einen axialen Schaftbereich (22) und eine Mehrzahl von um den Schaftbereich (22) regelmässig angeordneten Lappenbereichen (23) definiert, so dass der Schaft (8) im wesentlichen aus der Vereinigung des axialen Schaftbereiches (22) und der Lappenbereiche (23) besteht, und
in jedem Lappenbereich die Schaftmantelfläche ein Paar von Flanken (24,25) aufweist, die sich im wesentlichen vom Schaftbereich (22) bis in Nähe eines dem Schaftbereich (22) abgewandten Endes (26) des Lappenbereiches (23) erstrecken und unter denen mit Bezug auf die genannte Drehrichtung (D) jeweils eine vorlaufende Flanke (24) und eine nachlaufende Flanke (25) definiert ist,
und, in einem Querschnitt des Schaftes (8) rechtwinklig zur Längsachse (L) betrachtet, jeder Lappenbereich (23) in bezug auf eine durch die Längsachse (L) gehende diametrale Ebene asymmetrisch ausgebildet ist, wobei ein der vorlaufenden Flanke (24) entsprechender Abschnitt der Umrisslinie (17) kürzer ist als ein der nachlaufenden Flanke (25) entsprechender Abschnitt der Umrisslinie (17), so dass der Lappenbereich (23) beim Verlauf des radialen Abstands von der Längsachse (L) entlang der Umrisslinie (17) ein sägezahnähnliches Profil mit durchschnittlich steilerem Anstieg der vorlaufenden Flanke (24) und flacherem Abfall der nachlaufenden Flanke (25) aufweist.

## Claims

1. Screw (1) made of biodegradable material for bone surgery purposes, having an elongate screw body (2) which extends, in the direction of a longitudinal axis (L) thereof, between a proximal (3) and a distal (4) end thereof, and in which:
an outer surface (5) of the screw body (2) is provided with an external thread (6) coaxial with respect to the longitudinal axis (L), which is intended for guiding and holding the screw (1) on bone material of a patient and defines a direction of rotation (D) about the longitudinal axis (L) for the screwing-in of the screw body (2) and the advancing of the distal end (4) into the bone material,
an elongate channel (7) coaxial with respect to the longitudinal axis (L) is provided in the screw body (2), which channel (7) is open at the proximal end (3) for receiving a shaft (8) of a screwdriver (9) intended for turning the screw (1),
the channel (7) has a channel wall (13), the shape of which is radially symmetrical about the longitudinal axis (L) in discrete, regular rotary steps and affords, in a cross-section of the screw body (2) at right angles to the longitudinal axis (L), a trace line (17) which is a closed contour line (17) radially symmetrical about a trace (14) of the longitudinal axis (L) in discrete, regular rotary steps,
the channel wall (13) defines an axial channel area (22) and a plurality of lobe areas (23) arranged uniformly about the channel area (22), so that the channel (7) consists substantially of the combination of the axial channel area (22) and the lobe areas (23), and
in each lobe area the channel wall (13) has a pair of flanks (24, 25) which extend substantially from the channel area (22) to the vicinity of an end (26) of the lobe area (23) remote from the channel area (22), and among which there is defined, with reference to said direction of rotation (D), a leading flank (24) and a trailing flank (25), respectively,
**characterized in that** the channel (7) extends into the area of the distal end (4) of the screw (1), and, when considered in a cross-section of the screw body (2) at right angles to the longitudinal axis (L), each lobe area (23) is formed asymmetrical with respect to a diametrical plane passing through the longitudinal axis (L), a section of the contour line (17) which corresponds to the leading flank (24) being shorter than a section of the contour line (17) which corresponds to the trailing flank (25), so that the lobe area (23) has a sawtooth-like profile of a radial distance of the contour line (17) from the longitudinal axis (19) running along the contour line (17) with, on the average, a steeper raise of the leading flank (24) and a flatter fall of the trailing flank (25).

2. Screw according to claim 1, **characterized in that** the channel (7) extends into the area of the distal end (4) of the screw (1), and in each lobe area (23) at least one of said flanks (24) exhibits, at least in the vicinity of said end (26) of the lobe area (23), a shape which is increasingly leading in said direction of rotation (D) as the radial distance from the longitudinal axis (L) increases.

3. Screw according to claim 1 or 2, **characterized in that** the flanks (24, 25) are twisted helically about the longitudinal axis (L) in said direction of rotation (D), in such a way that, in two cross-sections of the screw body (2) at right angles to the longitudinal axis (L) which are different from one another, the contour lines (17) represent displacement images of each other which can be made congruent by plain rotation about the trace of the longitudinal axis (L).

4. Screw according to claim 1 or 2, **characterized in that** the flanks (24, 25) are tapered in the direction from the proximal (3) to the distal (4) end, in such a way that, in two cross-sections of the screw body (2) at right angles to the longitudinal axis (L) which are different from one another, the contour lines (17) represent similitude images of each other which can be made congruent by homothetic transformation about the trace (14) of the longitudinal axis (L).

5. Screw according to claim 3 and 4, **characterized in that** the flanks (24, 25) are both twisted and tapered, in such a way that, in two cross-sections of the screw body (2) at right angles to the longitudinal axis (L) which are different from one another, the contour lines (17) represent of each other similitude images which can be made congruent by a combination of plain rotation and homothetic transformation about the trace (14) of the longitudinal axis (L).

6. Screw according to any of claims 4 and 5, **characterized in that** the tapering in the direction from the proximal (3) to the distal (4) end corresponds to a semi-aperture angle of up to 10° and preferably of approximately 2°.

7. Screw according to claim 1 or 2, **characterized in that** in each lobe area (23) the two flanks (24, 25) extend at a distance from one another which tapers towards the end (26) of the lobe area (23) as the radial distance from the longitudinal axis (L) increases.

8. Screw according to claim 7, **characterized in that** the two flanks (24, 25) run together at the end (26) of the lobe area (23) to form an edge.

9. Screw according to claim 1 or 2, **characterized in that** in each lobe area (23), at least in the vicinity of the end (26) of the lobe area (23) the trailing flank (25) has a convex curvature facing said direction of rotation (D).

10. Screw according to claim 1, **characterized in that** in each lobe area (23), at least in the vicinity of the end (26) of the lobe area (23) the leading flank (24) is curved convexly with respect to said direction of rotation (D) or is substantially located in a plane passing through the longitudinal axis (L).

11. Screw according to claim 2, **characterized in that** in each lobe area (23), at least in the vicinity of the end (26) of the lobe area (23) the leading flank (24) is curved concavely with respect to said direction of rotation (D).

12. Screw according to claim 1 or 2, **characterized in that**, in a cross-section of the screw body (2) at right angles to the longitudinal axis (L), the contour line (17) corresponds substantially to a star-shaped broken line that is radially symmetrical about the trace (14) of the longitudinal axis (L) and has 3 to 8, and preferably 6, teeth or lobes (23) distorted for bias towards said direction of rotation (D).

13. Screw according to claims 2 and 12, **characterized in that** the teeth or lobes (23) are approximately sickle-shaped.

14. Screw according to claim 2, **characterized in that** in each lobe area (23) the two flanks (24, 25) extend at an approximately constant distance from one another.

15. Screw according to claim 14, **characterized in that**, in a cross-section of the screw body (2) at right angles to the longitudinal axis (L), the contour line (17) corresponds substantially to a bucketwheel-shaped broken line that is radially symmetrical about the trace (14) of the longitudinal axis (L) and has 3 to 8, and preferably 6, lobes (23) which are shaped approximately as ring segments of approximately constant width distorted for bias towards said direction of rotation (D).

16. Screw according to claim 2, **characterized in that** in each lobe area (23), in a cross-section of the screw body (2) at right angles to the longitudinal axis (L), a trace line (24) of the leading flank (24) is substantially rectilinear and inclined for bias towards to said direction of rotation (D).

17. Screw according to claim 16, **characterized in that** the inclination of the leading flank (24) is up to 10° and preferably about 5°.

18. Screwdriver (9) for driving a screw (1) according to claim 1 into bone material of a patient during bone surgery, the screwdriver (9) being provided with a grip (10) and, secured thereon, an elongate shaft (8) that can be introduced into and removed from the channel (7) of the screw (1), there being defined a direction of rotation (D) about the longitudinal axis (L) for the screwing-in of the screw (1) into the bone material, whereby
the shaft (8) has a shaft lateral surface, the shape of which is radially symmetrical about the longitudinal axis (L) in discrete, regular rotary steps and affords, in a cross-section of the screw body (2) at right angles to the longitudinal axis (L), a trace line (17) which is a closed contour line (17) radially symmetrical about a trace (14) of the longitudinal axis (L) in discrete, regular rotary steps,
the shaft lateral surface defines an axial shaft area (22) and a plurality of lobe areas (23) arranged uniformly about the shaft area (22), so that the shaft (8) substantially consists of the combination of the axial shaft area (22) and the lobe areas (23), and
in each lobe area the shaft lateral surface has a pair of flanks (24, 25) which extend substantially from the shaft area (22) to the vicinity of an end (26) of the lobe area (23) remote from the shaft area (22), and among which there is defined, with reference to said direction of rotation (D), a leading flank (24) and a trailing flank (25), respectively,
and, when considered in a cross-section of the shaft (8) at right angles to the longitudinal axis (L), each lobe area (23) is formed asymmetrical with respect to a diametrical plane passing through the longitudinal axis (L), a section of the contour line (17) which corresponds to the leading flank (24) being shorter than a section of the contour line (17) which corresponds to the trailing flank (25), so that the lobe area (23) has a sawtooth-like profile of a radial distance of the contour line (17) from the longitudinal axis (19) when considered along the contour line (17) with, on the average, a steeper raise of the leading flank (24) and a flatter fall of the trailing flank (25).

## Revendications

1. Vis (1) en matériau biodégradable pour la chirurgie osseuse, présentant un corps de vis allongé (2) qui s'étend dans la direction d'un axe longitudinal (L) de celui-ci entre une extrémité proximale (3) et une extrémité distale (4) de celui-ci, et dans laquelle:
une surface extérieure (5) du corps de vis (2) est munie d'un filetage externe (6) coaxial à l'axe longitudinal (L) et qui est destiné à guider et maintenir la vis (1) dans du matériau osseux d'un patient et définit une direction de rotation (D) autour de l'axe longitudinal (L) pour le vissage du corps de vis (2) et l'avance de l'extrémité distale (4) dans le matériau osseux,
dans le corps de vis (2) est prévu un canal allongé (7) coaxial à l'axe longitudinal (L) et qui est ouvert en son extrémité proximale (3) pour recevoir une tige (8) d'un tournevis (9) destiné à faire tourner la vis (1),
le canal (7) comporte une paroi de canal (13) dont la forme présente autour de l'axe longitudinal (L) une symétrie radiale avec des pas de rotation discrets réguliers et produit dans une section transversale du corps de vis (2) perpendiculaire à l'axe longitudinal (L) une trace (17) qui est une ligne de contour fermée (17) présentant autour d'une trace (14) de l'axe longitudinal (L) une symétrie radiale avec des pas de rotation discrets réguliers,
la paroi de canal (13) définit une région axiale de canal (22) et une pluralité de régions de lobe (23) uniformément réparties autour de la région de canal (22), de sorte que le canal (7) est constitué pour l'essentiel de la combinaison de la région axiale de canal (22) et des régions de lobe (23), et
dans chaque région de lobe, la paroi de canal (13) comporte une paire de flancs (24, 25) qui s'étendent pour l'essentiel depuis la région de canal (22) jusqu'au voisinage d'une extrémité (26) de la région de lobe (23) éloignée de la région de canal (22), et parmi lesquels sont respectivement définis, par rapport à ladite direction de rotation (D), un flanc avant (24) et un flanc arrière (25).
**caractérisée en ce que** le canal (7) s'étend jusque dans la région de l'extrémité distale (4) de la vis (1) et chaque région de lobe (23), considérée dans une section transversale du corps de vis (2) perpendiculaire à l'axe longitudinal (L), présente une forme dissymétrique par rapport à un plan diamétral passant par l'axe longitudinal (L), tandis qu'une section de la ligne de contour (17) qui correspond au flanc avant (24) est plus courte qu'une section de la ligne de contour (17) qui correspond au flanc arrière (25), de manière que la région de lobe (23) présente un profil en dents de scie de la distance radiale de la ligne de contour (17) à l'axe longitudinal (L) courant le long de la ligne de contour (17) avec, en moyenne, un pente ascendante plus raide du flanc avant (24) et une pente descendante plus douce du flanc arrière (25).

2. Vis selon la revendication 1, **caractérisée en ce que** le canal (7) s'étend jusque dans la région de l'extrémité distale (4) de la vis (1) et dans chaque région de lobe (23) au moins un desdits flancs (24) présente, au moins au voisinage de ladite extrémité (26) de la région de lobe (23), une forme dont l'avance dans ladite direction de rotation (D) s'accroît lorsque la distance radiale depuis l'axe longitudinal (L) augmente.

3. Vis selon la revendication 1 ou 2, **caractérisée en ce que** les flancs (24, 25) présentent une torsion hélicoïdale autour de l'axe longitudinal (L) dans ladite direction de rotation (D), de manière que dans deux sections transversales du corps de vis (2) perpendiculaires à l'axe longitudinal (L) et qui diffèrent l'une de l'autre, les lignes de contour (17) représentent des figures de déplacement l'une de l'autre qui peuvent être amenées en congruence par simple rotation autour de la trace de l'axe longitudinal (L).

4. Vis selon la revendication 1 ou 2, **caractérisée en ce que** les flancs (24, 25) présentent une diminution dans la direction allant de l'extrémité proximale (3) vers l'extrémité distale (4), de manière que dans deux sections transversales du corps de vis (2) perpendiculaires à l'axe longitudinal (L) et qui diffèrent l'une de l'autre, les lignes de contour (17) représentent des figures de similitude l'une de l'autre qui peuvent être amenées en congruence par homothétie autour de la trace (14) de l'axe longitudinal (L).

5. Vis selon les revendications 3 et 4, **caractérisée en ce que** les flancs (24, 25) présentent aussi bien une torsion hélicoïdale qu'une diminution, de manière que dans deux sections transversales du corps de vis (2) perpendiculaires à l'axe longitudinal (L) et qui diffèrent l'une de l'autre, les lignes de contour (17) représentent des figures de similitude l'une de l'autre qui peuvent être amenées en congruence par une combinaison de simple rotation et d'homothétie autour de la trace (14) de l'axe longitudinal (L).

6. Vis selon l'une des revendications 4 et 5, **caractérisée en ce que** la diminution dans la direction allant de l'extrémité proximale (3) vers l'extrémité distale (4) correspond à un demi-angle au sommet valant jusqu'à 10° et de préférence environ 2°.

7. Vis selon la revendication 1 ou 2, **caractérisée en ce que** dans chaque région de lobe (23) les deux flancs (24, 25) s'étendent à une distance l'un de l'autre qui diminue vers l'extrémité (26) de la région de lobe (23) lorsque la distance radiale depuis l'axe longitudinal (L) augmente.

8. Vis selon la revendication 7, **caractérisée en ce que** les deux flancs (24, 25) se rejoignent en formant une arête à l'extrémité (26) de la région de lobe (23).

9. Vis selon la revendication 1 ou 2, **caractérisée en ce que** dans chaque région de lobe (23), au moins au voisinage de l'extrémité (26) de la région de lobe (23) le flanc arrière (25) présente une courbure convexe vers ladite direction de rotation (D).

10. Vis selon la revendication 1, **caractérisée en ce que** dans chaque région de lobe (23), au moins au voisinage de l'extrémité (26) de la région de lobe (23) le flanc avant (24) présente une courbure convexe vers ladite direction de rotation (D) ou est pour l'essentiel situé dans un plan passant par l'axe longitudinal (L).

11. Vis selon la revendication 2, **caractérisée en ce que** dans chaque région de lobe (23), au moins au voisinage de l'extrémité (26) de la région de lobe (23) le flanc avant (24) présente une courbure concave vers ladite direction de rotation (D).

12. Vis selon la revendication 1 ou 2, **caractérisée en ce que**, dans une section transversale du corps de vis (2) perpendiculaire à l'axe longitudinal (L), la ligne de contour (17) correspond pour l'essentiel à une ligne brisée en forme d'étoile présentant autour de la trace (14) de l'axe longitudinal (L) une symétrie radiale avec 3 à 8 et de préférence 6 dents ou lobes (23) déformés en biais vers ladite direction de rotation (D).

13. Vis selon les revendications 2 et 12, **caractérisée en ce que** les dents ou lobes (23) sont approximativement en forme de faucille.

14. Vis selon la revendication 2, **caractérisée en ce que** dans chaque région de lobe (23) les deux flancs (24, 25) s'étendent à distance approximativement constante l'un de l'autre.

15. Vis selon la revendication 14, **caractérisée en ce que**, dans une section transversale du corps de vis (2) perpendiculaire à l'axe longitudinal (L), la ligne de contour (17) correspond pour l'essentiel à une ligne brisée en forme de roue à aubes présentant autour de la trace (14) de l'axe longitudinal (L) une symétrie radiale avec 3 à 8 et de préférence 6 lobes (23) approximativement en forme de segments annulaires de largeur approximativement constante déformés en biais vers ladite direction de rotation (D).

16. Vis selon la revendication 2, **caractérisée en ce que** dans chaque région de lobe (23), dans une section transversale du corps de vis (2) perpendiculaire à l'axe longitudinal (L) une trace (24) du flanc avant (24) est pour l'essentiel rectiligne et inclinée en biais vers ladite direction de rotation (D).

17. Vis selon la revendication 16, **caractérisée en ce que** l'inclinaison du flanc avant (24) vaut jusqu'à 10° et de préférence environ 5°.

18. Tournevis (9) pour visser une vis (1) selon la revendication 1 dans un matériau osseux d'un patient pendant une opération de chirurgie osseuse, le tournevis (9) comportant une poignée (10) et, fixée à celle-ci, une tige allongée (8) qui peut être introduite dans et retirée d'un canal (7) de la vis (1), une direction de rotation (D) autour d'un axe longitudinal (L) de la tige (8) étant définie pour le vissage de la vis (2) dans le matériau osseux, et dans lequel:
la tige (8) présente une surface latérale de tige dont la forme présente autour de l'axe longitudinal (L) une symétrie radiale avec des pas de rotation discrets réguliers et produit dans une section transversale de la tige (8) perpendiculaire à l'axe longitudinal (L) une trace (17) qui est une ligne de contour fermée (17) présentant autour d'une trace (14) de l'axe longitudinal (L) une symétrie radiale avec des pas de rotation discrets réguliers,
la surface latérale de tige définit une région axiale de tige (22) et une pluralité de régions de lobe (23) uniformément réparties autour de la région de tige (22), de sorte que la tige (8) est constituée pour l'essentiel de la combinaison de la région axiale de tige (22) et des régions de lobe (23), et
dans chaque région de lobe, la surface latérale de tige comporte une paire de flancs (24, 25) qui s'étendent pour l'essentiel depuis la région de tige (22) jusqu'au voisinage d'une extrémité (26) de la région de lobe (23) éloignée de la région de tige (22), et parmi lesquels sont respectivement définis, par rapport à ladite direction de rotation (D), un flanc avant (24) et un flanc arrière (25),
et chaque région de lobe (23), considérée dans une section transversale de la tige (8) perpendiculaire à l'axe longitudinal (L), présente une forme dissymétrique par rapport à un plan diamétral passant par l'axe longitudinal (L), tandis qu'une section de la ligne de contour (17) qui correspond au flanc avant (24) est plus courte qu'une section de la ligne de contour (17) qui correspond au flanc arrière (25), de manière que la région de lobe (23) présente un profil en dents de scie de la distance radiale de la ligne de contour (17) à l'axe longitudinal (L) courant le long de la ligne de contour (17) avec, en moyenne, un pente ascendante plus raide du flanc avant (24) et une pente descendante plus douce du flanc arrière (25).
